(19) Europäisches Patentamt / European Patent Office / Office européen des brevets

(11) **EP 4 570 837 A1**

(12) **EUROPEAN PATENT APPLICATION**
published in accordance with Art. 153(4) EPC

(43) Date of publication:
**18.06.2025 Bulletin 2025/25**

(21) Application number: **23852648.7**

(22) Date of filing: **10.08.2023**

(51) International Patent Classification (IPC):
***C08F 220/28*** (2006.01) ***A61K 9/51*** (2006.01)
***A61K 47/22*** (2006.01) ***A61K 47/32*** (2006.01)
***A61K 49/12*** (2006.01) ***A61K 49/18*** (2006.01)
***C07D 257/02*** (2006.01)

(52) Cooperative Patent Classification (CPC):
**A61K 9/51; A61K 47/22; A61K 47/32; A61K 49/12; A61K 49/18; C07D 257/02; C08F 220/28**

(86) International application number:
**PCT/JP2023/029358**

(87) International publication number:
**WO 2024/034686 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.08.2022 JP 2022128538**

(71) Applicant: **KOWA COMPANY, LTD.**
**Naka-ku**
**Nagoya-shi**
**Aichi 460-8625 (JP)**

(72) Inventors:
- **CHIDA, Tsukasa**
  **Higashimurayama-shi, Tokyo 189-0022 (JP)**
- **FUJIMAKI, Nobuhiro**
  **Higashimurayama-shi, Tokyo 189-0022 (JP)**
- **ECHIGO, Marina**
  **Higashimurayama-shi, Tokyo 189-0022 (JP)**

(74) Representative: **Wächtershäuser & Hartz Patentanwaltspartnerschaft mbB**
**Weinstraße 8**
**80333 München (DE)**

(54) **POLYMER CONTRAST AGENT**

(57) The present invention relates to a copolymer in which a chelating agent molecule is bonded to a copolymer X comprising structural units of (A), (B), and (C).

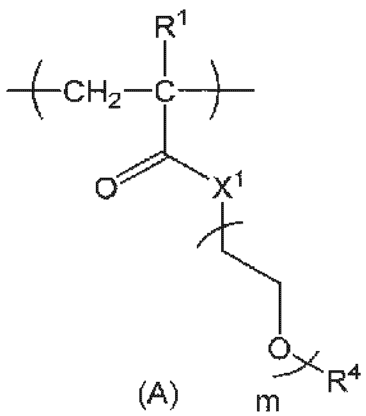
(A) ,

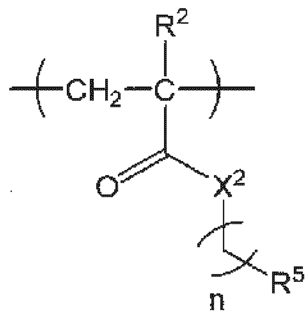
(B) ,

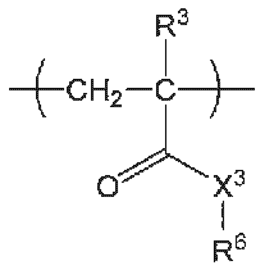
(C)

$R^1$, $R^2$, and $R^3$ are the same or different and represent hydrogen or $C_{1-3}$ alkyl, $R^4$ represents $C_{1-3}$ alkyl, $R^5$ represents hydrogen, $C_{1-18}$ alkyl, 3- to 8- membered cycloalkyl optionally having substituent, adamantyl, $C_{6-18}$ aryl optionally having





**(Cont. next page)**

substituent, or 5- to 10- membered heteroaryl group optionally having substituent, $X^1$, $X^2$, and $X^3$ are the same or different and represent oxygen, sulfur, or N-$R^7$, $R^6$ represents hydrogen, leaving group, or linker, $R^7$ represents hydrogen or $C_{1-3}$ alkyl group, m represents 1 to 100, and n represents 0 to 3.

## Description

Technical Field

The present invention relates to a polymer contrast agent useful for nuclear magnetic resonance imaging. More specifically, the present invention relates to a paramagnetic metal-containing polymer contrast agent.

Background Art

In recent years, research on drug delivery systems (DDS) have been energetically conducted as a technique for efficiently and safely delivering a drug to disease sites. Among them, there is an increasing demand for DDS employing nanoparticles as drug delivery carriers as a technique for enhancing the selectivity of drug accumulation by utilizing the structural characteristics of the disease site.

Despite innovative advances in treatment and diagnostic techniques, cancer still accounts for a large proportion of noncommunicable disease-related deaths. With the widespread use of molecular targeted therapy using antibody drugs, there has been active development of in vitro diagnostic drugs for purpose of testing malignant tumorrelated genes. However, there are still cancers which are difficult to find at an early stage, and thus it is desired to further improve diagnosis accuracy by utilizing various diagnosis modalities.

Although there are various known diagnostic imaging techniques for cancer, magnetic resonance imaging (MRI) is an excellent tomographic imaging method that is free from effects of radiation exposure and has high spatial resolution. Imaging methods by the MRI are roughly divided into contrast MRI using a contrast agent and simple MRI without the contrast-enhanced agent. Gadolinium (Gd), which is a paramagnetic material, is often used as the contrast agent in the contrast MRI. Because Gd is a metal of a rare earth element, Gd has strong toxicity in the body, and thus a compound in which Gd is complexed with a chelating agent has been used.

In differentiation of diseases using the contrast-enhanced MRI, a contrast enhancement effect of the contrast agent and a temporal change thereof are utilized.

Since an existing contrast agent is diffused into the whole body immediately after administration and then excreted, it has a limited period of time in the imaging, and it is difficult to achieve a remarkable effect in imaging of tumors other than those in the brain. Therefore, the contrast agent is used for purpose of auxiliary data acquisition. Under the circumstances, gadoxetate disodium (trade name: EOB·Primovist Injection Syringes, manufactured and sold by Bayer Holding Ltd.) imparts a specific uptake effect to normal hepatocytes by converting a substituent of the chelating agent to an aromatic ring. This effect prolongs a retention time of the contrast agent in a normal liver tissue, and thus can be applied to differentiation of liver tumors. However, this is not applicable for differentiation of tumors of other organs. As such, an MRI contrast agent capable of differentiating tumors of various organs in a wide range has not yet been put into practical use.

Under the circumstances, development of pharmaceutical products to which nanotechnology is applied has been actively discussed. In solid cancer tissue, since the structure of a neovascular vessel (tumor blood vessel) is immature as compared to a normal blood vessel, a cell gap of about several hundred nm is generated in the vascular endothelium, which allows the permeation of a large amount of substance. Due to this structural feature, it is known that a polymeric compound containing nanoparticles selectively permeates a tumor blood vessel and accumulates in solid cancer tissue. Furthermore, in solid cancer tissue, a lymphatic system involved in excretion of polymers malfunctions, so that permeated nanoparticles are continuously retained in the tissue (Enhanced permeability and retention effect, EPR effect). Since a general low molecular drug leaks out of a blood vessel due to membrane permeation of vascular cells, it is non-selectively distributed in tissue and does not accumulate in solid cancer tissue. According to the methodology of the EPR effect, nanoparticle-based drug delivery results in improved tissue selectivity to solid cancer in the distribution of a drug, since distribution to tissue is governed by the permeability of vascular endothelial cell gaps. Therefore, the EPR effect is a promising academic support in the development of nanotechnology-applied medicines (nanomedicine) targeting solid cancer.

It is believed that a drug delivery process in the EPR effect occurs through blood flow and that the extravasation process of nanoparticles is passive. Therefore, to maximize the accumulation of nanoparticles in solid cancer, it is important to impart a molecular design that can withstand long-term blood retention to the constituent components of nanoparticles used as drug delivery carriers. Drug delivery carriers are therefore required to have the ability to avoid barriers such as non-specific interactions with blood components, foreign body recognition by the reticuloendothelial system (RES) in the liver, spleen, and lungs, and glomerular filtration in the kidneys. In addition, it is known that these barriers can be overcome by optimizing particle properties such as particle diameter and surface modification with a biocompatible polymer. For example, it is desirable that the particle diameter of the drug delivery carrier be greater than about 6 nm, which is the threshold for renal clearance, and less than 200 nm, which may avoid recognition by the RES.

The particle diameter of the drug delivery carrier is also known to affect tissue permeation at a disease site. For example, the anticancer activity of drug-encapsulating nanoparticles with particle diameters measuring 30 nm, 50 nm, 70

nm, and 100 nm, which exhibit equivalent blood retention, has been compared and studied, and it has been revealed that drug-encapsulating nanoparticles with a particle diameter of 30 nm reach the deep parts of a disease site and thus exhibit the highest therapeutic effect (Non-Patent Literature 1). Therefore, it would be desirable for the particle diameter of nanoparticles for a drug delivery carrier targeting solid cancer to be as small as possible within the range that avoids renal clearance.

As nanoparticles for drug delivery carriers, methods using colloidal dispersions such as liposomes, emulsions, or nanoparticles, methods using biologically derived raw materials such as albumin, methods using natural polymers such as natural polysaccharides, or methods using synthetic polymers, have been developed. Among them, synthetic polymers are widely used as constituents of drug delivery carriers because it is possible to prepare nanoparticles whose particle diameter is precisely controlled by appropriately selecting monomers as constituent components and synthesis methods.

For example, a method for utilizing an amphiphilic block copolymer composed of a hydrophilic segment and a hydrophobic segment as a drug delivery carrier is disclosed. The block copolymer spontaneously associates in an aqueous medium by, for example, hydrophobic interaction between molecules as a driving force to form core-shell type nanoparticles (polymeric micelles). It is known that it is possible to encapsulate a low molecular drug in or bind it to the hydrophobic segment of the polymeric micelle, and the obtained drug-encapsulating polymeric micelle exhibits high blood stability, and due to selective accumulation in solid cancer through the EPR effect, high anticancer activity compared the administration of a solution of a low molecular drug (Patent Literature 1). However, since the polymeric micelle is an assembly of multiple molecules, a particle diameter of about 30 nm is the lower limit value that can be prepared. Also in application to an MRI contrast agent, for example, a method in which iron microparticles coated with polysaccharides are used (Non-Patent Literature 2), a method in which a paramagnetic metal complex is contained in a liposome (Patent Literature 2), and a method in which a paramagnetic metal complex is bonded to a synthetic polymer (block copolymer) to form a polymeric micelle (Patent Literature 3) have been developed. However, in any method, the lower limit of the particle diameter of each of the nanoparticles is about 20 nm, and it has not been achieved to finely control the particle diameter around 10 nm where influence of renal clearance can be avoided.

Meanwhile, among nanoparticles formed of a synthetic polymer, those which form particles by, for example, chemical crosslinking, hydrophobic interaction, or ionic bond within a single chain as a driving force (hereinafter abbreviated as single chain nanoparticles (SCNPs)) are known to form nanoparticles having a small particle diameter of 20 nm or less (Non-Patent Literature 2). Therefore, although SCNPs are expected to be useful as drug delivery carriers, a technique for precisely controlling their particle diameter has not been found so far.

Citation List

Patent Literature

Patent Literature 1: JP 3270592 B
Patent Literature 2: JP 5883797 B
Patent Literature 3: JP 4892378 B

Non-Patent Literature

Non-Patent Literature 1: H. Cabral et al., Nat. Nanotechnol. 6 815-823 (2011)
Non-Patent Literature 2: Avnesh S. Thakor et al., J Nucl Med.57,1833-1837 (2016)
Non-Patent Literature 3: Jose A. Pomposo, Single-Chain Polymer Nanoparticles: Synthesis, Characterization, Simulations, and Applications (2017)

Summary of Invention

Technical Problem

An object of the present invention is to provide a new polymer contrast agent, and more specifically, an MRI polymer contrast agent capable of widely differentiating tumors of various organs.

Solution to Problem

To achieve the above-mentioned objects, the present inventors conducted extensive research and discovered that a terpolymer of an acrylic acid derivative has the property of forming SCNPs in water. Furthermore, the present inventors succeeded in creating a novel polymer for a drug delivery carrier, which is capable of precisely controlling the particle diameter of SCNPs at a minute scale of 20 nm or less and about 10 nm, and has high tumor accumulation. Further, the present inventors also succeeded in creation of a derivative of the terpolymer that forms a contrast agent molecule-bonded SCNP in which contrast agent molecules are loaded into the polymer. As the contrast agent molecule-bonded SCNP was administered to a model mouse subcutaneously transplanted with a colon cancer, it exhibited an excellent imaging ability.

The present invention relates to the following inventions.

[1] A copolymer (hereinafter, also referred to as a chelating agent-bonded copolymer) in which a chelating agent molecule is bonded to a copolymer X comprising structural units represented by the following formulas (A), (B), and (C).

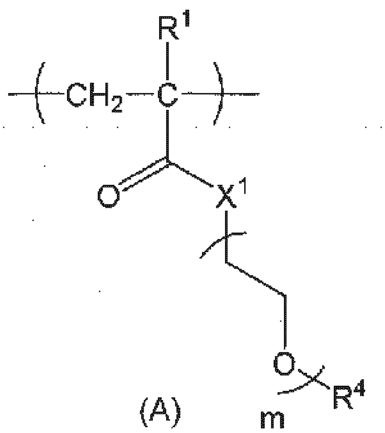
(A) ,
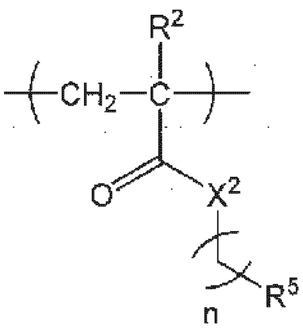
(B) ,
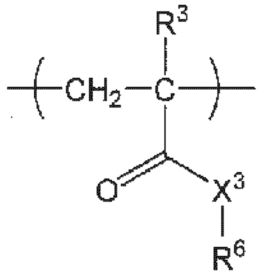
(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker; $R^7$ represents a hydrogen atom or a $C_{1-3}$ alkyl group, m represents an integer of 1 to 100, and n represents an integer of 0 to 3.

[2] The copolymer according to [1], wherein the copolymer X is a copolymer formed by polymerization of three types of monomers represented by the following general formulas (1) to (3):

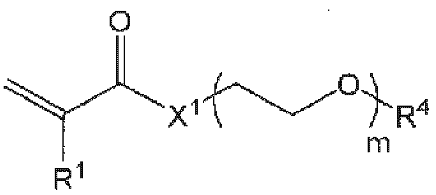
,
(1)
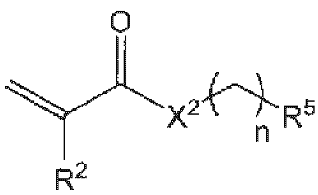
,
(2)
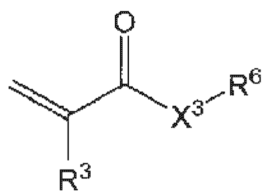
(3)

wherein, $R^1$, $R^2$, and $R^3$ are the same or different and represent a hydrogen atom or a $C_{1-3}$ alkyl group; $R^4$ represents a $C_{1-3}$ alkyl group; $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent; $X^1$, $X^2$, and $X^3$ are the same or different and represent an oxygen atom, a sulfur atom, or N-$R^7$; $R^6$ represents a hydrogen atom, a leaving group, or a linker; $R^7$ represents a hydrogen atom or a $C_{1-3}$ alkyl group; m represents an integer of 1 to 100; and n represents an integer of 0 to 3.

[3] The copolymer according to [1] or [2], wherein $R^1$ is a hydrogen atom.

[4] The copolymer according to any one of [1] to [3], wherein $R^2$ is a hydrogen atom.

[5] The copolymer according to any one of [1] to [4], wherein $R^3$ is a hydrogen atom.

[6] The copolymer according to any one of [1] to [5], wherein $R^4$ is a methyl group.
[7] The copolymer according to any one of [1] to [6], wherein $R^5$ is a $C_{6-18}$ aryl group optionally having a substituent.
[8] The copolymer according to any one of [1] to [7], wherein $R^5$ is a phenyl group.
[9] The copolymer according to any one of [1] to [8], wherein $R^6$ is a hydrogen atom.
[10] The copolymer according to any one of [1] to [8], wherein the leaving group of $R^6$ is a group represented by the following formula (4).

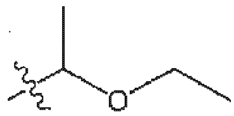

(4)

[11] The copolymer according to any one of [1] to [8], wherein the linker of $R^6$ is one selected from the following formulas (5) to (7).

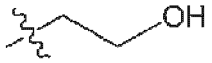

(5) ,

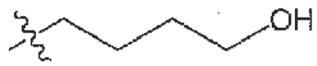

(6) ;

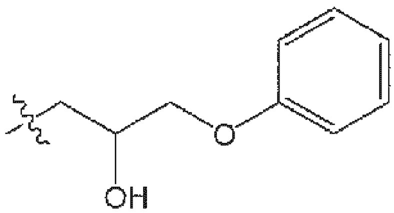

(7)

[12] The copolymer according to any one of [1] to [11], wherein $X^1$ is an oxygen atom.
[13] The copolymer according to any one of [1] to [12], wherein $X^2$ is an oxygen atom.
[14] The copolymer according to any one of [1] to [13], wherein $X^3$ is an oxygen atom.
[15] The copolymer according to any one of [1] to [14], wherein m is an integer of 4 to 22.
[16] The copolymer according to any one of [1] to [15], wherein n is 1.
[17] The copolymer according to [1] to [16], wherein a ratio of the structural units (A), (B), and (C) is 0.01 to 100 parts by mass of (B) and 0.1 to 100 parts by mass of (C) with respect to 1 part by mass of (A).
[18] The copolymer according to any one of [2] to [16], wherein 0.01 to 100 parts by mass of monomer (2) and 0.1 to 100 parts by mass of monomer (3) are polymerized with respect to 1 part by mass of monomer (1).
[19] The copolymer according to any one of [1] to [18], wherein the copolymer has a number average molecular weight of 5 000 to 150 000.
[20] The copolymer according to any one of [1] to [19], wherein the chelating agent molecule is a molecule having a residue represented by the following formula (a):

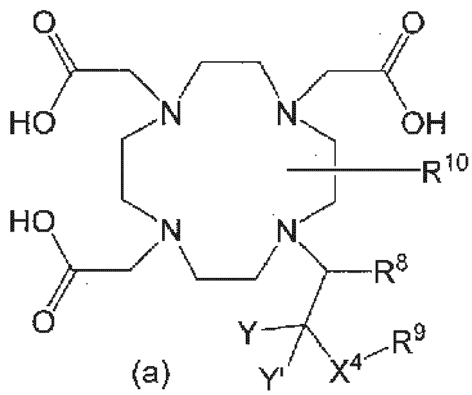

(a)

wherein, $R^8$ represents a hydrogen atom or a hydroxyalkyl group; $R^9$ and $R^{10}$ represent a hydrogen atom or $-[(CH_2)_o-L-(CH_2)_p]-*$; $X^4$ represents an oxygen atom, a sulfur atom, $N-R^7$, or $-CH_2-O-$; Y and Y' represent a hydrogen atom, a methyl group, or a hydroxy group, or Y and Y' together represent an oxygen atom; L represents an arylene group, a cycloalkylene group, -S-S-, -C(=O)O-, -OC(=O)-, -C(=O)NH-, -NHC(=O)-, -NHC(=O)O-, -OC(=O)NH-, or a

peptide bond including 1 to 4 amino acid residues; * represents a bond to the copolymer; and o and p independently represent an integer of 0 to 10, and where $R^9$ is a hydrogen atom, $R^{10}$ is $-[(CH_2)_o-L-(CH_2)_p]-*$, and where $R^9$ is $-[(CH_2)_o-L-(CH_2)_p]-*$, $R^{10}$ is a hydrogen atom.

[21] The copolymer according to any one of [1] to [19], wherein the chelating agent molecule is 1,4,7,10-tetraazacyclododecane-1,4,7,10-tetraacetic acid (DOTA), 1-(2-hydroxypropyl)-1,4,7,10-tetraazacyclododecane-1,2,3-triacetic acid (HP-DO3A), 10-[1,1,1-tris(hydroxymethyl)methyl]-1,4,7-triscarboxymethyl-1,4,7,10-tetraazacyclododecane (DO3A-butrol), 1,4,7,10-tetraazacyclododecane-1,4,7-triacetic acid-10-(2-thioethyl)acetamide (DO3A-Thiol), or S-2-(4-aminobenzyl)-1,4,7,10-1,4,7,10-tetraazacyclododecane-1,4,7,10-tetraacetic acid (DOTA-p-$NH_2$-Bn).

[22] The copolymer according to any one of [1] to [21], wherein binding of the chelating agent molecule to the copolymer X is a covalent bond or a non-covalent bond.

[23] A polymer contrast agent (hereinafter, also referred to as a contrast agent molecule-bonded copolymer) including the copolymer according to any one of [1] to [22] and a paramagnetic metal.

[24] The polymer contrast agent according to any one of [1] to [23], wherein the paramagnetic metal is gadolinium or manganese.

[25] A diagnostic imaging drug including the copolymer according to any one of [1] to [22] and a paramagnetic metal.

[26] The diagnostic imaging drug according to any one of [1] to [23], wherein the paramagnetic metal is gadolinium or manganese.

[27] A single chain nanoparticle including the copolymer according to any one of [1] to [22].

[28] A pharmaceutical composition including the copolymer according to any one of [1] to [25].

[29] A single chain nanoparticle including the polymer contrast agent according to [23] or [24].

[30] A single chain nanoparticle including the diagnostic imaging drug according to [25] or [26].

Advantageous Effects of Invention

As will be apparent from examples described later, the polymer contrast agent in which the contrast agent molecules are loaded into SCNP obtained by self-association of the copolymer of the present invention can be applied as a diagnostic imaging drug for malignant tumors since the polymer contrast agent showed a temporal change in the imaging ability in a C26-transplanted, tumor-bearing mouse. Further, since the polymer contrast agent of the present invention has high imaging ability at a low dose, it is possible to provide a diagnostic imaging drug for malignant tumors capable of achieving both action enhancement and side effect suppression.

Brief Description of Drawings

Fig. 1 is a diagram showing a $^1H$-NMR spectrum of a copolymer obtained in Example 1, measured by using nuclear magnetic resonance (NMR).

Fig. 2 is a diagram showing a chromatogram of the copolymer obtained in Example 1, obtained by gel permeation chromatography (GPC).

Fig. 3 is a diagram showing a $^1H$-NMR spectrum of a copolymer obtained in Example 69, measured by using the nuclear magnetic resonance (NMR).

Fig. 4 is a diagram showing a chromatogram of the copolymer obtained in Example 69, obtained by gel permeation chromatography (GPC).

Fig. 5 is a diagram showing a UV spectrum of a copolymer obtained in Example 98, measured by using ultraviolet spectrum measurement (UV).

Fig. 6 is a diagram showing a $^1H$-NMR spectrum of a chelating agent-bonded copolymer obtained in Example 106, measured by using the nuclear magnetic resonance (NMR).

Fig. 7 is a diagram showing a $^1H$-NMR spectrum of a chelating agent-bonded copolymer obtained in Example 134, measured by using the nuclear magnetic resonance (NMR).

Fig. 8 is a diagram showing a $^1H$-NMR spectrum of a chelating agent-bonded copolymer obtained in Example 142, measured by using the nuclear magnetic resonance (NMR).

Fig. 9 is a diagram showing a $^1H$-NMR spectrum of a chelating agent-bonded copolymer obtained in Example 150, measured by using the nuclear magnetic resonance (NMR).

Fig. 10 is a diagram showing a $^1H$-NMR spectrum of a chelating agent-bonded copolymer obtained in Example 151, measured by using the nuclear magnetic resonance (NMR).

Fig. 11 is a diagram showing a particle diameter measurement result (scattering intensity distribution) of a Gd-DOTA-bonded SCNP obtained in Example 152 in dynamic light scattering (DLS).

Fig. 12 is a diagram showing a temporal change in the imaging ability of the Gd-DOTA-bonded SCNP obtained in Example 152 in a contrast test on the C26-transplanted, tumor-bearing mouse.

Description of Embodiments

Terms in the present description are used in the meanings commonly used in the field unless otherwise specified. Hereinafter, the present invention will be described in more detail.

In the present description, the term "nanoparticle" refers to a structure having a particle diameter of 100 nm or less.

In the present description, the term "single chain nanoparticle (SCNP)" refers to a nanoparticle formed by using, for example, chemical crosslinking, hydrophobic interaction, or ionic bonding in a single chain as a driving force. The SCNP often indicates a nanoparticle having a relatively small particle diameter of 20 nm or less among nanoparticles.

In the present description, the term "initiator" means an initiator for thermal radical polymerization such as an azo compound or a peroxide.

In the present specification, the term "chain transfer agent" refers to a compound that causes a chain transfer reaction in radical polymerization, and is preferably a compound having a thiocarbonyl group.

In the present description, the term "$C_{1-3}$ alkyl group" means a linear or branched, alkyl group having 1 to 3 carbon atoms, and examples thereof include a methyl group, an ethyl group, an n-propyl group, and an isopropyl group.

In the present description, the term "$C_{1-18}$ alkyl group" means a linear or branched, alkyl group having 1 to 18 carbon atoms, and examples thereof include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

In the present description, the term "3- to 8-membered cycloalkyl group optionally having a substituent" means a cyclic alkyl group having 3 to 8 carbon atoms, and examples thereof include a cyclopropyl group, a cyclobutyl group, a cyclopentyl group, a cyclohexyl group, a cycloheptyl group, and a cyclooctyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "$C_{6-18}$ aryl group optionally having a substituent" means a monocyclic or polycyclic condensed aromatic hydrocarbon group, and examples thereof include a phenyl group, a naphthyl group, an anthracenyl group, a phenanthrenyl group, a triphenylenyl group, a pyrenyl group, a chrysenyl group, and a naphthacenyl group. Further, a "$C_{6-14}$ aryl group optionally having a substituent" means a monocyclic or polycyclic condensed aromatic hydrocarbon group, and examples thereof include a phenyl group, a naphthyl group, an anthracenyl group, and a phenanthrenyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "5- to 10-membered heteroaryl group optionally having a substituent" means a 5- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a furyl group, a thienyl group, a pyrrolyl group, a pyridinyl group, a pyrazinyl group, a pyrimidinyl group, a pyridazinyl group, an imidazolyl group, a pyrazyl group, a thiallyl group, an oxazolyl group, an isoxazolyl group, a 1,3,4-thiadiazolyl group, a 1,2,3-triazolyl group, a 1,2,4-triazolyl group, and a tetrazolyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The term "6- to 10-membered heteroaryl group optionally having a substituent" means a 6- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as the atoms constituting the ring. Examples of the monocyclic aromatic heterocyclic group include a pyridinyl group, a pyrazinyl group, a pyrimidinyl group, and a pyridazinyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group,

and an isoquinolinyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, examples of the "halogen atom" include a fluorine atom, a chlorine atom, a bromine atom, and an iodine atom.

In the present description, the "hydroxyalkyl group" means a linear or branched, alkyl group having 1 to 3 carbon atoms in which one hydrogen atom is substituted with a hydroxyl group, and examples thereof include a hydroxymethyl group, a hydroxyethyl group, and a hydroxypropyl group.

In the present description, the "arylene group" means a divalent aromatic hydrocarbon cyclic group having 6 to 10 carbon atoms, and examples thereof include phenylene and naphthylene.

In the present description, the "cycloalkylene group" means a divalent cyclic saturated hydrocarbon group having 3 to 7 carbon atoms, and examples thereof include cyclopropylene, cyclobutylene, cyclopentylene, cyclohexylene, and cycloheptylene.

In the present description, the "contrast agent" is a compound to be administered into the body for purpose of improving accuracy of diagnostic imaging, and is a drug used in, for example, the MRI, computed tomography (CT), positron emission tomography (PET), single photon emission computed tomography (SPECT), or ultrasonic diagnostic imaging. The contrast agent is usually a compound (contrast agent molecule) in which the paramagnetic metal is coordinated to respective chelating agent molecules. The contrast agent molecule may be loaded into the copolymer by an action such as electrostatic interaction, hydrogen bond, hydrophobic interaction, or covalent bond to the copolymer X of the present invention.

In the present description, the "chelating agent molecule" is a compound that forms a complex by a coordination bond to a metal ion. The metal ion is preferably a paramagnetic metal ion.

In the present description, the "pharmaceutical composition" means one composed of the copolymer (hereinafter, also referred to as the chelating agent-bonded copolymer or the contrast agent molecule-bonded copolymer) in which the chelating agent molecule or the contrast agent molecule is bonded to (loaded into due to an action such as electrostatic interaction, hydrogen bond, hydrophobic interaction, or covalent bond) the copolymer X of the present invention, and a carrier. Examples of the loading form of the contrast agent molecule include a form in which the contrast agent molecule is present on the particle surface, a form in which the contrast agent molecule is contained in a nanoparticle, and a combination form thereof where the contrast agent molecule-bonded copolymer forms the nanoparticle.

One embodiment of the present invention is a copolymer (chelating agent molecule-bonded copolymer or contrast agent molecule-bonded copolymer) in which a chelating agent molecule or a contrast agent molecule is bonded to a copolymer X having structural units represented by the following formulas (A), (B), and (C):

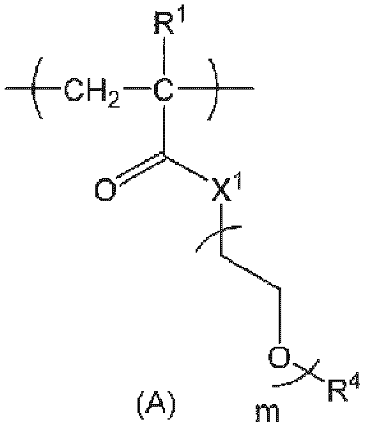
(A) ,
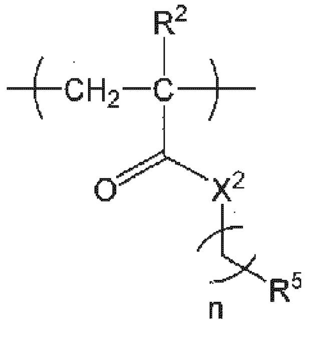
(B) ,
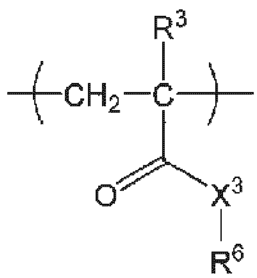
(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

In the copolymer X of the present invention, the structural unit (A) functions as a unit that imparts hydrophilicity, and the structural unit (B) functions as a unit that imparts hydrophobicity. Further, the structural unit (C) functions as a scaffold to which an active ingredient (drug or contrast agent molecule) is bonded to the copolymer X, the chelating agent molecule-bonded copolymer, or the contrast agent molecule-bonded copolymer. Having these three structural units serves to imparting the copolymer X, the chelating agent molecule-bonded copolymer, or the contrast agent molecule-bonded copolymer of the present invention a property of forming SCNP in water, and to facilitating the formed SCNP to precisely control particle diameter at a minute scale of 20 nm or less, and to function as a drug delivery carrier having high tumor accumulation.

$R^1$ in the structural unit (A) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^1$ represents an oxygen atom, the sulfur atom, or N-$R^7$, and is preferably an oxygen atom, a sulfur atom, or NH, and more preferably an oxygen atom.

m represents an integer of 1 to 100, preferably an integer of 3 to 100, and from a viewpoint of imparting good hydrophilicity, preferably 3 to 80, more preferably from 4 to 60, still more preferably 4 to 40, and yet more preferably from 4 to 22.

$R^4$ represents a $C_{1-3}$ alkyl group, specifically a methyl group, an ethyl group, an n-propyl group or an isopropyl group, preferably a methyl group or an ethyl group, and more preferably a methyl group.

$R^2$ in the structural unit (B) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^2$ represents an oxygen atom, a sulfur atom, or N-$R^7$, and is preferably an oxygen atom, a sulfur atom, or NH, and more preferably an oxygen atom.

n represents an integer of 0 to 3, preferably an integer of 1 to 3, and more preferably 1.

$R^5$ represents a hydrogen atom, a $C_{1-18}$ alkyl group, a 3-to 8-membered cycloalkyl group optionally having a substituent, an adamantyl group, a $C_{6-18}$ aryl group optionally having a substituent, or a 5- to 10-membered heteroaryl group optionally having a substituent, and from a viewpoint of imparting the hydrophobicity to the structural unit (B), preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, more preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and still more preferably a $C_{1-18}$ alkyl group, a 3- to 8-membered cycloalkyl group, an adamantyl group, or a $C_{6-18}$ aryl group. Meanwhile, a 3- to 8-membered cycloalkyl group optionally having a substituent, an adamantyl group, a $C_{6-14}$ aryl group optionally having a substituent, or a 6- to 10-membered heteroaryl group optionally having a substituent is also preferable. Here, the substituent is preferably one or more types selected from a halogen atom, an alkyl group having 1 to 6 carbon atoms, an alkenyl group having 2 to 6 carbon atoms, and an alkynyl group having 2 to 6 carbon atoms.

$R^3$ in the structural unit (C) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^3$ represents an oxygen atom, a sulfur atom, or N-$R^7$, and is preferably an oxygen atom, a sulfur atom, or NH, and more preferably an oxygen atom.

$R^6$ represents a hydrogen atom, a leaving group, or a linker. The leaving group is a group that can detach when the structural unit (C) binds to the drug (contrast agent molecule) or the chelating agent molecule, and the linker is a group that can be used for crosslinking when the structural unit (C) binds to the drug (contrast agent molecule) or the chelating agent molecule. As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, the linker is preferably a group having a functional group such as a hydroxyl group, an amino group, a thiol group, or a carboxyl group as a substituent.

Preferred examples of the leaving group of $R^6$ include a group represented by the following formula (4):

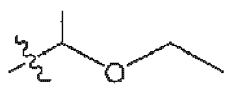

(4)

Preferred examples of the linker of $R^6$ include groups selected from the following formulas (5) to (7).

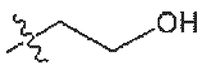

(5)

,

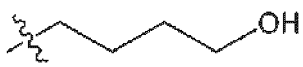

(6)

,

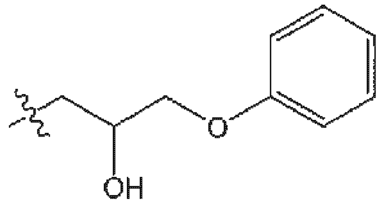

(7)

No text.

The copolymer X of the present invention is the copolymer having the structural units represented by formulas (A), (B) and (C). The copolymer X may be a random copolymer or a block copolymer and is preferably a random copolymer. As for a composition ratio of each structural unit in one molecule, when (A) is 1 part by mass, preferably (B) is 0.01 to 100 parts by mass and (C) is 0.1 to 100 parts by mass; more preferably (B) is 0.05 to 18 parts by mass and (C) is 0.1 to 20 parts by mass, and particularly preferably (B) is 0.05 to 4 parts by mass and (C) is 0.1 to 16 parts by mass.

A polymerization degree of the copolymer X of the present invention is not particularly limited, and the number average molecular weight thereof is preferably 5 000 to 150 000, and more preferably 8 000 to 150 000.

In the copolymer of the present invention, as described above, the monomer represented by general formula (1) functions as a unit that imparts the hydrophilicity, and the monomer represented by general formula (2) functions as a unit that imparts the hydrophobicity. Further, the monomer represented by general formula (3) functions as a scaffold to which the drug and the copolymer are bonded. Examples of the monomer functioning as the hydrophobic unit represented by general formula (2) include monomers represented by the following formulas.

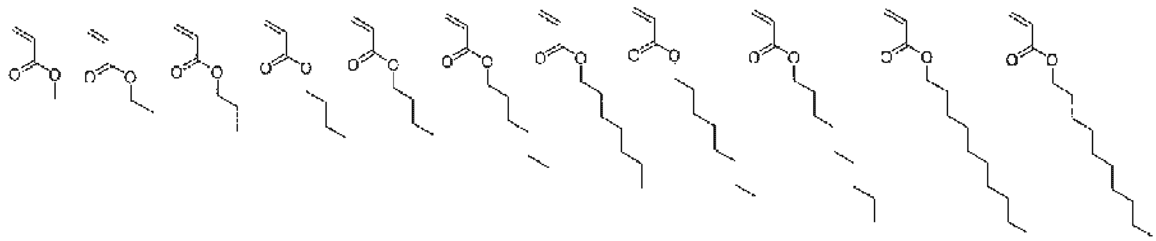

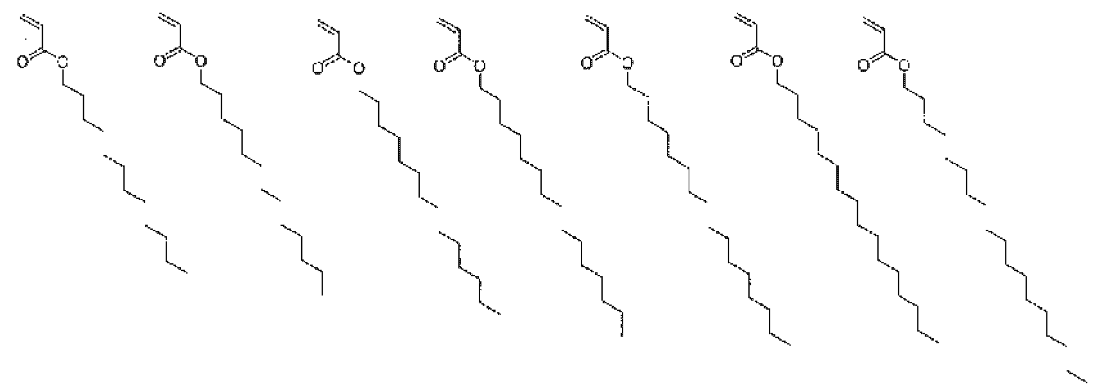

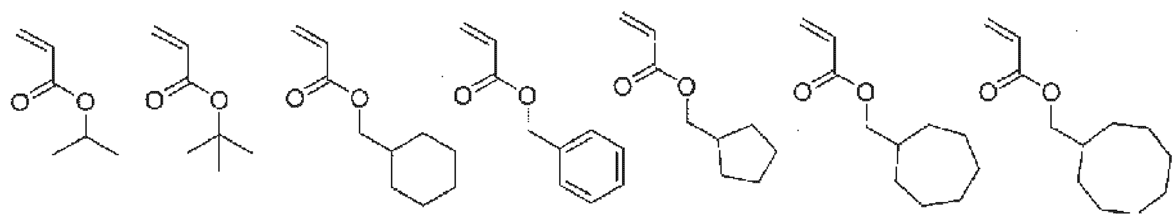

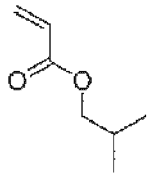 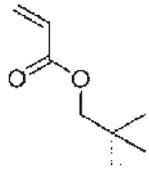 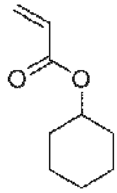 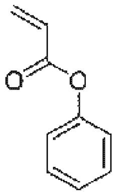 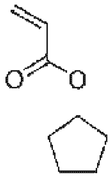 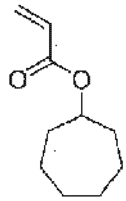 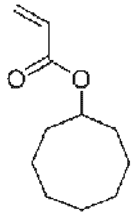

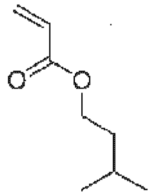 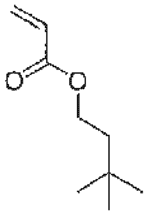 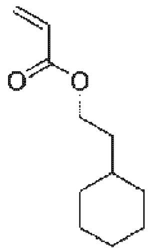 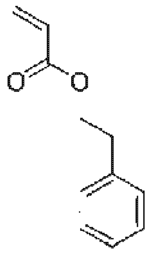 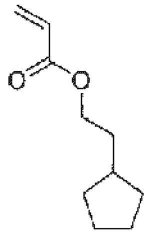 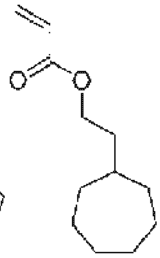 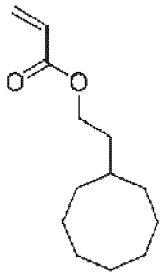

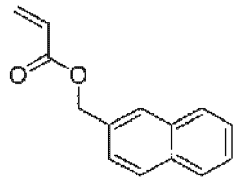 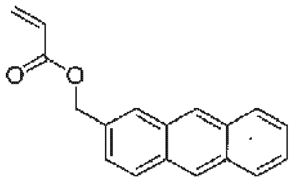 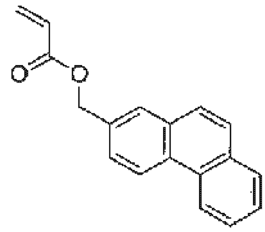 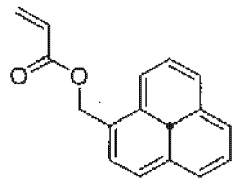

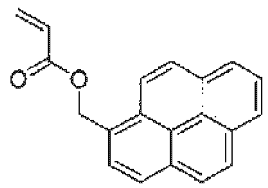 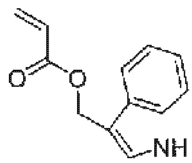 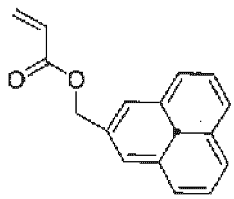 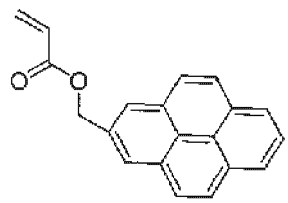

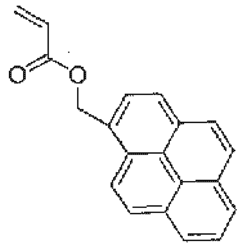 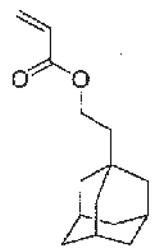

No text.

In general formula (1), $R^1$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (2), $R^2$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (3), $R^3$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (1), $R^4$ represents a $C_{1-3}$ alkyl group, specifically a methyl group, an ethyl group, an n-propyl group or an isopropyl group, preferably a methyl group or an ethyl group, and more preferably a methyl group.

In general formula (1), $X^1$ represents an oxygen atom, a sulfur atom, or N-$R^7$, and is preferably an oxygen atom, a sulfur atom, or NH, and more preferably an oxygen atom.

In general formula (1), m represents an integer of 1 to 100, and is preferably an integer of 3 to 100, and preferably 3

to 80, more preferably 4 to 60, still more preferably 4 to 40, and yet more preferably 4 to 22 from the viewpoint of imparting good hydrophilicity.

In general formula (2), $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and from the viewpoint of imparting hydrophobicity to the structural unit (B), a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is preferable, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is more preferable, and a $C_{1-18}$ alkyl group, a 3- to 8-membered cycloalkyl group, an adamantyl group, or a $C_{6-18}$ aryl group is still more preferable. In addition, a 3- to 8-membered cycloalkyl group optionally having a substituent, an adamantyl group, a $C_{6-14}$ aryl group optionally having a substituent, or a 6- to 10-membered heteroaryl group optionally having a substituent is also preferable. Here, the substituent is preferably one or more selected from a halogen atom, an alkyl group having 1 to 6 carbon atoms, an alkenyl group having 2 to 6 carbon atoms, and an alkynyl group having 2 to 6 carbon atoms.

In general formula (2), $X^2$ represents an oxygen atom, a sulfur atom, or N-$R^7$, and is preferably an oxygen atom, a sulfur atom, or NH, and more preferably an oxygen atom.

In general formula (2), n represents an integer of 0 to 3, preferably an integer of 1 to 3, and more preferably 1.

In general formula (3), $R^6$ represents a hydrogen atom, a leaving group, or a linker. As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of $R^6$ include a group represented by the following formula (4):

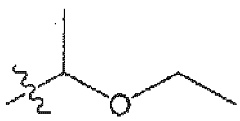

(4)

Preferred specific examples of the linker of $R^6$ include groups selected from the following formulas (5) to (7):

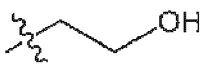

(5)

,

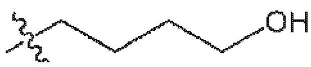

(6)

,

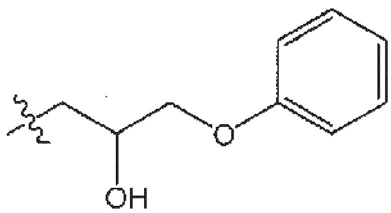

(7)

No text.

In general formula (3), $X^3$ represents an oxygen atom, a sulfur atom, or N-$R^7$, and is preferably an oxygen atom, a sulfur atom, or NH, and more preferably an oxygen atom.

The copolymer X of the present invention is formed by copolymerizing three monomers represented by general formulas (1) to (3). The copolymerization may be random copolymerization or block copolymerization, and those formed by random copolymerization are preferable. As for a blending ratio of the three monomers, it is preferable that 0.01 to 100 parts by mass of monomer (2) and 0.1 to 100 parts by mass of monomer (3) are polymerized, it is more preferable that 0.05 to 18 parts by mass of monomer (2) and 0.1 to 20 parts by mass of monomer (3) are polymerized, and it is particularly preferable that 0.05 to 4 parts by mass of monomer (2) and 0.1 to 16 parts by mass of monomer (3) are polymerized, with respect to 1 part by mass of monomer (1).

In addition, "solvates" in which various solvents are coordinated are also included in the copolymer X of the

present invention. In the present description, examples of the "solvate" include a hydrate and an ethanolate. The solvent may be coordinated to the copolymer X of the present invention in any number.

The copolymer X of the present invention can be produced by various known methods. The production method is not particularly limited, and for example, the copolymer X can be produced according to a basic polymer synthesis method described below.

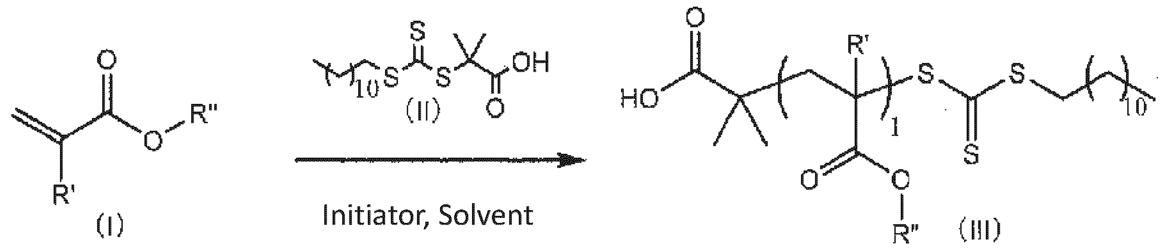


In the formula, R' represents a hydrogen atom or a $C_{1-3}$ alkyl group, and R" represents a group represented by $R^4$, $R^5$ or $R^6$.

This reaction shows a step of producing a polymer (III) by reacting a monomer (I) with a chain transfer agent (II) and an initiator. This reaction can be performed in the absence of a solvent or in a solvent such as alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate, and it is preferable to use aromatic hydrocarbons such as toluene and xylene as a solvent. As the chain transfer agent, for example, it is possible to use 2-(dodecylthiocarbonothioylthio)-2-methylpropionic acid (DDMAT), cyanomethyl dodecyltrithiocarbonate (CDTTC), 2-cyano-2-propyldodecyl trithiocarbonate (CPDTTC), 4-cyano-4-[(dodecylsulfanylthiocarbonyl)sulfanyl]pentanoic acid (CDSPA), or 2-(dodecylthiocarbonothioylthio)-2-methylpropionic acid 3-azido-1-propanol ester ($N_3$-CTA), and it is preferable to use DDMAT. Where polymerization is carried out by using a chain transfer agent, the copolymer X of the present invention has a structure in which a part or all of a structure of the chain transfer agent is partially bonded. Where the copolymer X includes a structure of the chain transfer agent, the structure may be removed by an appropriate method. As the initiator, an azo polymerization initiator such as 2,2'-azobisisobutyronitrile (AIBN), 1,1'-azobis(cyclohexanecarbonitrile) (ACHN), 2,2'-azobis-2-methylbutyronitrile (AMBN), 2,2'-azobis-2,4-dimethylvaleronitrile (ADVN), or dimethyl 2,2'-azobis(2-methylpropionate) (MAIB) can be used, and AIBN is preferably used. The reaction temperature is 0 to 300°C, preferably 0 to 150°C, and more preferably 1 to 100°C, and the reaction time is 1 minute to 48 hours, and preferably 5 minutes to 24 hours. In this reaction, a random copolymerized copolymer X can be produced by carrying out the reaction in the coexistence of monomers (I) having different structures.

The chelating agent bonded to the copolymer X is a molecule having a residue represented by the following formula (a):

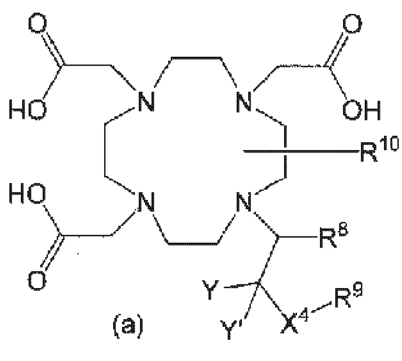

wherein, $R^8$ represents a hydrogen atom or a hydroxyalkyl group; $R^9$ and $R^{10}$ represent a hydrogen atom or $-[(CH_2)_o\text{-L-}(CH_2)_p]$-*; $X^4$ represents an oxygen atom, a sulfur atom, N-$R^7$, or $-CH_2$-O-; Y and Y' represent a hydrogen atom, a methyl group, or a hydroxy group, or Y and Y' together represent an oxygen atom; L represents an arylene group, a cycloalkylene group, -S-S-, -C(=O)O-, -OC(=O)-, -C(=O)NH-, -NHC(=O)-, -NHC(=O)O-, -OC(=O)NH-, or a peptide bond including 1 to 4 amino acid residues; * represents a bond to the copolymer; and o and p independently represent an integer of 0 to 10, and where $R^9$ is a hydrogen atom, $R^{10}$ is $-[(CH_2)_o\text{-L-}(CH_2)_p]$-*, and where $R^9$ is $-[(CH_2)_o\text{-L-}(CH_2)_p]$-*, $R^{10}$ is a hydrogen atom.]

Examples of the chelating agent include 1,4,7,10-tetraazacyclododecane-1,4,7,10-tetraacetic acid (DOTA),

1,4,7,10-tetraazacyclododecane-1,4,7-triacetic acid (DO3A), 1-(2-hydroxypropyl)-1,4,7,10-tetraazacyclododecane-1,2,3-triacetic acid (HP-DO3A), 10-[1,1,1-tris(hydroxymethyl)methyl]-1,4,7-triscarboxymethyl-1,4,7,10-tetraazacyclododecane (DO3A-butrol), 1,4,7,10-tetraazacyclododecane-N,N',N'',N'''-tetraacetic acid mono-(N-hydroxysuccinimidyl) ester (DOTA-NHS), [(2S,5S,8S,11S)-4,7-bis-carboxymethyl-2,5,8,11-tetramethyl-1,4,7,10-tetraazacyclo-dodecan-1-yl]acetic acid (M4DO3A), [(2S,5S,8S,11S)-4,7,10-tris-carboxymethyl-2,5,8,11-tetramethyl-1,4,7,10- tetraazacyclododecane-1-yl]acetic acid (M4DOTA), α,α',α",α'''-tetramethyl-1,4,7,10-tetraazacyclododecane-1,4,7,10-tetraacetic acid (DOTMA), (R)-2-[(2S,5S,8S,11S)-4,7,10-tris-((R)-1-carboxyethyl)-2,5,8,11-tetramethyl-1,4,7,10-tetraazacyclododecane-1-yl]propionic acid (M4DOTMA), 1,4,7,10-tetraazacyclododecane-1,4,7-triacetic acid-10-(2-thioethyl)acetamide (DO3A-Thiol), S-2-(4-aminobenzyl)-1,4,7,10-1,4,7,10-tetraazacyclododecane-1,4,7,10-tetraacetic acid (p-$NH_2$-Bn-DOTA), 2-methyl-1,4,7,10-tetraazacyclododecane-1,4,7,10-tetraacetic acid (MCTA), diethylenetriaminepentaacetic acid (DTPA), 4-carboxy-5,8,11-tris(carboxymethyl)-1-phenyl-2-oxa-5,8,11-triazatridecane-13-oic acid (BOPTA), ethylenediaminetetraacetic acid (EDTA), 3,6,9,15-tetraazabicyclo[9.3.1]pentadeca-1(15),11,13-triene-3,6,9-triacetic acid (PCTA12), 1,4,8,11-tetraazacyclotetradecane-N,N',N",N'''-tetraacetic acid (TETA), 1,4,7,10-tetraazacyclotridecane-N,N',N",N'''-tetraacetic acid (TRITA), 1,5,9,13-tetraazacyclohexadecane-N,N',N",N'''-tetraacetic acid (HETA), 10-phosphonomethyl-1,4,7,10-tetraazacyclododecane-1,4,7-triacetic acid (MPDO3A), N,N'-bis(2-hydroxybenzyl)-ethylenediamine-diacetic acid (HBED), N,N'-bis-(2-hydroxyphenylglycine)-ethylenediamine (EHPG), 2-[bis[2-[carboxylatomethyl-[2-(2-methoxyethylamino)-2-oxoethyl]amino]ethyl]amino]acetate (DTPA-BMEA), N-[2-[bis(carboxymethyl)amino]-3-(4-ethoxyphenyl)propyl]-N-[2-[bis(carboxymethyl)amino]ethyl]glycine (EOB-DTPA), N,N-bis[2-[bis(carboxymethyl)amino]ethyl]-L-glutamic acid (DTPA-Glu), N,N-bis[2-[bis(carboxymethyl)amino]ethyl]-L-lysine (DTPA-Lys), N,N-bis[2-[carboxymethyl[(methylcarbamoyl)methyl]amino]-ethyl]glycine (DTPA-BMA), and (+/-)-trans-3,10,13,19-tetraazatricyclo[13.3.1.$0^{4,9}$]nonadeca-1(19),15,17-triene-3,10,13-triacetic acid (cyclo-PCTA12).

Examples of the paramagnetic metal ion include a gadolinium ion, a manganese ion, an iron ion, a nickel ion, a cobalt ion, a dysprosium ion, and a terbium ion, and among the ions, the gadolinium ion or the manganese ion is preferable.

The contrast agent molecule-bonded copolymer of the present invention includes the chelating agent-bonded copolymer and the paramagnetic metal. As the contrast agent molecule, a known MRI contrast agent can be used, and a complex molecule of the paramagnetic metal ion and formula (a) is preferable, Gd-DOTA, Gd-HP-DO3A, Gd-DO3A-butrol, Gd-DO3A-Thiol, and Gd-DOTA-p-NH2-Bn are more preferable, and Gd-DOTA is particularly preferable.

A salt of the contrast agent molecule-bonded copolymer of the present invention is not particularly limited as long as the salt is a pharmaceutically acceptable salt. Examples of such salt include alkali metal salts such as a sodium salt and a potassium salt, salts with metals of Group 2 elements such as a calcium salt and a magnesium salt, organic amine salts such as a phenethylamine salt, and ammonium salts.

Where geometric isomers or optical isomers are present in the contrast agent molecule-bonded copolymer of the present invention, mixtures or separations of those isomers are also included in the scope of the present invention. Separation of the isomers can be performed by a conventional method.

The contrast agent molecule-bonded copolymer of the present invention can be produced by various known methods. The production method is not particularly limited, and for example, the contrast agent molecule-bonded copolymer can be produced according to a synthesis method described below.

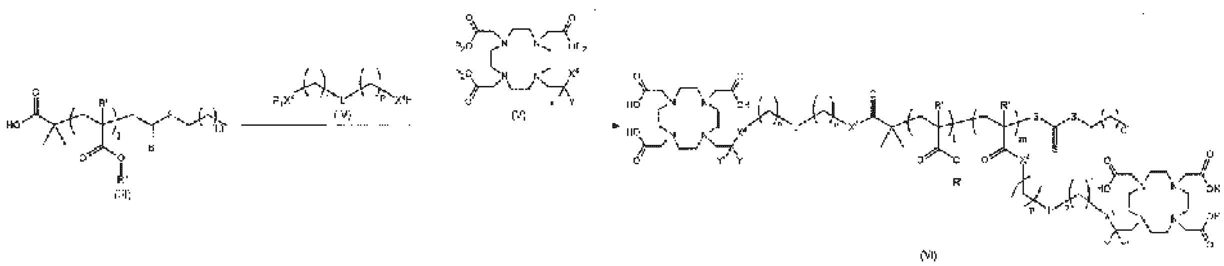

In the formula, R', R", $X^4$, Y, Y', or L represents a similar group as described above, and $P_1$ or $P_2$ represents a protecting group.

This reaction refers to a process of producing a chelating agent-bonded copolymer (VI) by reacting the copolymer (III) with a linker (IV) and a chelating agent (V) in a solvent or without a solvent, in the presence of a condensing agent and in the presence or absence of a reaction accelerator.

The solvent is not particularly limited, and this reaction can be performed in a solvent, for example, alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate. It is preferable to use an aprotic polar solvent such as tetrahydrofuran, dichloromethane, N,N-dimethylformamide, N,N-dimethylacetamide, N-methylpyrrolidone, acetonitrile, or ethyl acetate.

The linker is not particularly limited as long as the linker can link the copolymer and the chelating agent, and examples thereof include ethylenediamine, N-Boc-ethylenediamine, 1,3-propanediamine, N-Boc-1,3-propanediamine, 1,4-butanediamine, N-Boc-1,4-butanediamine, 1,5-pentanediamine, N-Boc-1,5-pentanediamine, 1,6-hexanediamine, N-Boc-1,6-hexanediamine, 1,7-heptanediamine, N-Boc-1,7-heptanediamine, 1,8-octanediamine, N-Boc-1,8-octanediamine, 1,9-nonanediamine, N-Boc-1,9-nonanediamine, 1,10-decanediamine, N-Boc-1,10-decanediamine, 2-aminoethanol, 2-(Boc-amino)-1-ethanol, 3-amino-1-propanol, 3-(Boc-amino)-1-propanol, 4-amino-1-butanol, 4-(Boc-amino)-1-butanol, 5-amino-1-pentanol, 5-(Boc-amino)-1-pentanol, 6-amino-1-hexanol, 6-(Boc-amino)-1-hexanol, 7-(Boc-amino)-1-heptanol, 8-(Boc-amino)-1-octanol, 9-(Boc-amino)-1-nonanol, 10-(Boc-amino)-1-decanol, glycolic acid, tert-butyl glycolate, 3-hydroxypropionic acid, tert-butyl 3-hydroxypropanoate, 4-hydroxybutyric acid, tert-butyl 4-hydroxybutanoate, cystamine, N-Boc-cystamine, 6-maleimidohexanoic acid N-succinimidyl ester, 4-maleimidobutyric acid N-succinimidyl ester, 3-maleimidopropionic acid N-succinimidyl ester, 3-(2-pyridyldithio)propionic acid N-succinimidyl ester, 4-(N-maleimidomethyl)cyclohexanecarboxylate N-succinimidyl ester, (S)-2-[(S)-2-amino-3-methylbutanamido]-N-[4-(hydroxymethyl)phenyl]-5-ureidopentanamide, [(S)-1-[[(S)-1-[[4-(hydroxymethyl)phenyl]amino]-1-oxo-5-ureidopentan-2-yl]amino]-3-methyl-1-oxobutan-2-yl]carbamic acid (9H-fluoren-9-yl)methyl ester, [(S)-1-[[(S)-1-[[4-(hydroxymethyl)phenyl]amino]-1-oxo-5-ureidopentan-2-yl]amino]-3-methyl-1-oxobutan-2-yl]carbamic acid allyl ester, 6-(2,5-dioxo-2,5-dihydro-1H-pyrrol-1-yl)-N-[(S)-1-[[(S)-1-[[4-(hydroxymethyl)phenyl]amino]-1-oxo-5-ureidopentan-2-yl]amino]-3-methyl-1-oxobutan-2-yl]hexanamide, β-alanine, β-alanine tert-butyl ester, and a pharmaceutically acceptable salt thereof, and N-Boc-ethylenediamine, 2-(Boc-amino)-1-ethanol, tert-butyl glycolate, and N-Boc-cystamine are preferable.

Where the chelating agent includes a functional group linkable to the copolymer X in its structure, it is also possible to directly link the copolymer and the chelating agent without interposing the linker.

The protecting group represented by $P_1$ and $P_2$ is not particularly limited, and can be selected with reference to, for example, a literature (Protective Groups in Organic Synthesis Fifth Edition, John Wiley & Sons, Inc.).

Examples of the condensing agent include carbodiimide reagents such as dicyclohexylcarbodiimide (DCC), 1-ethyl-3-(3-dimethylaminopropyl)carbodiimide (EDC), 1-ethyl-3-(3-dimethylaminopropyl)carbodiimide hydrochloride (WSC-HCl), and diisopropylcarbodiimide (DIPCDI), and phosphonium salt type or guanidinium salt type reagents such as (1H-benzotriazol-1-yloxy)tris(dimethylamino)phosphonium hexafluorophosphate (BOP), (1H-benzotriazol-1-yloxy)tris(pyrrolidino)phosphonium hexafluorophosphate (PyBOP), 1-[bis(dimethylamino)methylene]-1H-1,2,3-triazolo(4,5-b)pyridinium-3-oxide hexafluorophosphate (HATU), (1-cyano-2-ethoxy-2-oxoethylideneaminooxy)dimethylaminomorpholinocarbenium hexafluorophosphate (COMU), chloro-N,N,N',N'-tetramethylformamidinium hexafluorophosphate (TCFH), 1-[bis(dimethylamino)methylene]-1H-benzotriazolium-3-oxide hexafluorophosphate (HBTU), 1-[bis(dimethylamino)methylene]-1H-benzotriazolium-3-oxide tetrafluoroborate (TBTU), 1-[bis(dimethylamino)methylene]-5-chloro-1H-benzotriazolium-3-oxide hexafluorophosphate (HCTU), and 1-[bis(dimethylamino)methylene]-5-chloro-1H-benzotriazolium-3-oxide tetrafluoroborate (TCTU), and among the reagents, 1-ethyl-3-(3-dimethylaminopropyl)carbodiimide hydrochloride (WSC-HCl), (1H-benzotriazol-1-yloxy)tris(pyrrolidino)phosphonium hexafluorophosphate (PyBOP), 1-[bis(dimethylamino)methylene]-1H-1,2,3-triazolo(4,5-b)pyridinium-3-oxodohexafluorophosphate (HATU), (1-cyano-2-ethoxy-2-oxoethylideneaminooxy)dimethylaminomorpholinocarbenium hexafluorophosphate (COMU), and chloro-N,N,N',N'-tetramethylformamidinium hexafluorophosphate (TCFH) are preferable.

Examples of the reaction accelerator include triethylamine, diisopropylethylamine, pyridine, lutidine, picoline, N,N-dimethylaminopyridine (DMAP), 1-methylimidazole, 2,2,6,6-tetramethylpiperidine (TMP), 7-methyl-1,5,7-triazabicyclo[4.4.0]dec-5-ene (MTBD), 1,5,7-triazabicyclo[4.4.0]dec-5-ene (TBD), N-methylmorpholine (NMM), 1,8-diazabicyclo[5.4.0]-7-undecene (DBU), 1-hydroxybenzotriazole (HOBt), 6-chloro-1-hydroxybenzotriazole (6-Cl-HOBt), 3,4-dihydro-3-hydroxy-4-oxo-1,2,3-benzotriazine (HOOBt), and 1-hydroxy-7-azabenzotriazole (HOAt), and among the reaction accelerators, diisopropylethylamine, N,N-dimethylaminopyridine (DMAP), 1-methylimidazole, 2,2,6,6-tetramethylpiperidine (TMP), and 1,8-diazabicyclo[5.4.0]-7-undecene (DBU) are preferable.

The reaction temperature may be from 0°C to 100°C, preferably from 1 to 80°C, and the reaction time may be from 5 minutes to 1 week, and preferably from 2 hours to 3 days. To smoothly advance the reaction, the reaction may be performed under a nitrogen stream or an argon stream.

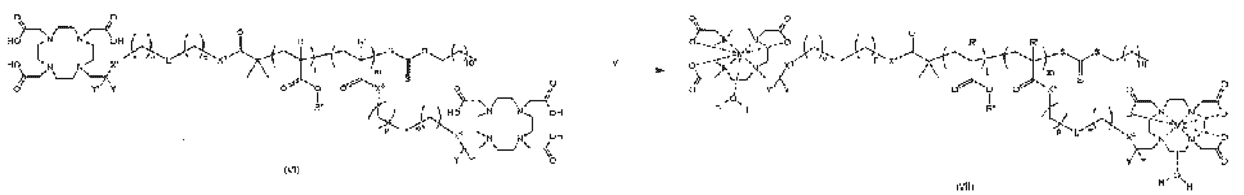

In the formula, R', R", $X^4$, Y, Y', or L represents a similar group as described above, and M represents a paramagnetic metal ion.

This reaction refers to a process of producing a contrast agent molecule-bonded copolymer (VII) by reacting a chelating agent-bonded polymer (VI) with a paramagnetic metal ion (M).

This reaction can be performed in water or in a solvent, for example, alcohols such as methanol, ethanol, 1-propanol, and 2-propanol; an aprotic polar solvent such as N,N-dimethylformamide, N,N-dimethylacetamide, N-methylpyrrolidone, acetonitrile, or ethyl acetate, and is preferably performed in water. The paramagnetic metal ion is not particularly limited as long as the ion has coordination ability to the chelating agent, and it is possible to use, for example, Fe(2+), Fe(3+), Cu(2+), Ni(2+), Rh(2+), Co(2+), Cr(3+), Gd(3+), Eu(3+), Dy(3+), Tb(3+), Pm(3+), Nd(3+), Tm(3+), Ce(3+), Y(3+), Ho(3+), Er(3+), La(3+), Yb(3+), Mn(3+), or Mn(2+), and it is preferable to use Gd(3+) or Mn(2+). The reaction temperature may be from 0 to 300°C, preferably from 0 to 150°C, and more preferably from 1 to 100°C, and the reaction time may be from 1 minute to 48 hours, and preferably from 5 minutes to 24 hours.

The produced polymer X and contrast agent molecule-bonded copolymer of the present invention can be purified by a polymer isolation and purification method generally known in the field of polymer chemistry. Specific examples thereof include treatment operations such as extraction, recrystallization, salting out using, for example, ammonium sulfate or sodium sulfate, centrifugation, dialysis, ultrafiltration, adsorption chromatography, ion exchange chromatography, hydrophobic chromatography, normal phase chromatography, reverse phase chromatography, gel filtration, gel permeation chromatography, affinity chromatography, electrophoresis, countercurrent distribution, and combinations thereof.

The copolymer X and the contrast agent molecule-bonded copolymer of the present invention can be utilized as carriers for transporting various drugs (contrast agents). For example, a pharmaceutical composition including the contrast agent molecule-bonded copolymer in which the contrast agent is loaded into (contained in) the copolymer X of the present invention can be used as a contrast agent and/or a diagnostic drug for various cancer diseases such as a colon cancer, a duodenal cancer, a gastric cancer, a pancreatic cancer, a liver cancer, a lung cancer, a uterine cancer, an ovarian cancer, and brain tumors because the tumor accumulation ability is high, as confirmed in test examples described later.

When the copolymer and the contrast agent molecule-bonded copolymer of the present invention are used as a drug transport carrier, the dose and the number of doses may be appropriately selected in consideration of, for example, administration form, age and body weight of a patient, and nature or severity of a symptom to be treated, and the dose and the number of doses should not be limited. However, when a polymer encapsulating a drug is intravenously injected by an injection, for example, for an adult (60 kg), a single dose is preferably administered in an amount of 0.12 mg to 12 000 000 mg, more preferably 1.2 mg to 1 200 000 mg, and particularly preferably 12 to 120 000 mg.

The pharmaceutical composition of the present invention can be produced by mixing the copolymer X of the present invention with the contrast agent molecule. Further, the pharmaceutical composition can also be produced by mixing the copolymer X of the present invention with the chelating agent molecule and then with the paramagnetic metal. Preferably, the single chain nanoparticle may be produced using the contrast agent molecule-bonded copolymer of the present invention, or the single chain nanoparticle of the copolymer X of the present invention may be produced and then mixed with the contrast agent molecule. The single chain nanoparticle can be produced by a known method.

In the pharmaceutical composition of the present invention, the contrast agent molecule may be loaded into the copolymer X or the contrast agent molecule-bonded copolymer by the action such as electrostatic interaction, hydrogen bond, hydrophobic interaction, or covalent bond.

A route of administration of the pharmaceutical composition of the present invention is desirably the most effective one for treatment, and the pharmaceutical composition can be administered by a parenteral administration preparation such as an oral administration preparation, an injection, or a transdermal administration preparation. For example, parenteral administration such as intraarterial injection, intravenous injection, subcutaneous injection, intramuscular injection, or intraperitoneal injection is preferable, and intraarterial injection and intravenous injection are more preferable. The number of doses should not be limited, and examples thereof include one to several doses per week average.

Various preparations suitable for the route of administration can be produced by a conventional method by appropriately selecting preparation additives such as excipients, extenders, binders, wetting agents, disintegrants, lubricants, surfactants, dispersants, buffers, preservatives, solubilizing agents, antiseptics, flavoring agents, soothing agents, stabilizers, and isotonizing agents that are conventionally used in formulation.

The preparation additives that can be contained in the various preparations described above are not particularly limited as long as the preparation additives are pharmaceutically acceptable. Examples of such preparation additives include purified water, water for injection, distilled water for injection, pharmaceutically acceptable organic solvents, collagen, polyvinyl alcohol, polyvinylpyrrolidone, carboxyvinyl polymer, sodium alginate, water-soluble dextran, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, and lactose. Additives to be used are appropriately selected according to various preparations, and can be used alone or in combination.

The injection can also be prepared as a non-aqueous diluent (for example, polyethylene glycol, vegetable oils such as olive oil, and alcohols such as ethanol), a suspension, or an emulsion. Sterilization of the injection can be performed by filtration sterilization using a filter, and blending of, for example, a microbicide. In addition, the injection can be

produced in a form of preparation before use. That is, the injection can be formed into a sterile solid composition by, for example, lyophilization, and can be dissolved in water for injection, distilled water for injection, or another solvent before use.

## Examples

Hereinafter, the present invention will be described more specifically with reference to examples. These examples are provided for purpose of exemplification and are not intended to limit embodiments of the invention.

### [Example 1] Production of poly[(benzyl acrylate)-co-(poly(ethylene glycol)methyl ether acrylate)-co-(1-ethoxyethyl acrylate)]

(1) Synthesis of 1-ethoxyethyl acrylate (EEA)

Ethyl vinyl ether (28.725 mL) was weighed under an argon atmosphere, and thereto was added a phosphoric acid (50 mg) under ice cooling. Thereto was added acrylic acid (17.15 mL), and the mixture was stirred at room temperature for 48 hours. Further thereto was added hydrotalcite (3 g), and the mixture was stirred for 2 hours, and the reaction was stopped. After celite filtration, unreacted ethyl vinyl ether was removed by evaporation. Thereto was added phenothiazine (up to 500 ppm) as a polymerization inhibitor, and the mixture was purified by distillation under reduced pressure together with calcium hydride (distillation temperature of 28 to 32°C). The obtained 1-ethoxyethyl acrylate was dispensed into a glass vial and stored at -30°C.

$^{13}$C NMR (400MHz, $CDCl_3$), $\delta$, ppm: 15.29 ($-OCH_2CH_3$), 21.16 ($-COOCH(CH_3)$), 64.98($-OCH_2-$), 96.73($-COOCH(CH_3)$), 128.84 ($CH_2CH-$), 131.43 ($CH_2CH-$), 166.00 (-COO).

(2) Synthesis of poly[(benzyl acrylate)-co-(poly(ethylene glycol) methyl ether acrylate)-co-(1-ethoxyethyl acrylate)]

100 mg of 2-(dodecylthiocarbonothioylthio)-2-methylpropionic acid (DDMAT) was weighed and dissolved in 17.3 mL of toluene to prepare a DDMAT/toluene stock solution (5.78 mg/mL as a DDMAT concentration). Similarly, 22 mg of 2,2'-azobis(2-methylpropionitrile) (AIBN) was weighed and dissolved in 17.3 mL of toluene to prepare an AIBN/toluene stock solution (AIBN concentration: 1.27 mg/mL). Separately, 1.296 g of poly(ethylene glycol) methyl ether acrylate (mPEGA, the average value (n) of the numbers of repetitions of ethylene glycol is 9), 0.394 g of benzyl acrylate (BnA), 0.039 g of 1-ethoxyethyl acrylate, 1.73 mL of a DDMAT/toluene stock solution, and 1.73 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 $\times$ g, 5 min) was repeated 3 times, and finally vacuum drying was performed to obtain 1.223 g of poly[(benzyl acrylate)-co-(poly(ethylene glycol) methyl ether acrylate)-co-(1-ethoxyethyl acrylate)].

As a result of analyzing the polymerization degree of each monomer and the number average molecular weight ($M_{n,NMR}$) from a $^1$H-NMR spectrum of the obtained copolymer measured using NMR, the polymerization degree of mPEGA (n = 9) was 102, the polymerization degree of BnA was 94, the polymerization degree of EEA was 9, and $M_{n,NMR}$ was 65 900. The molecular weight dispersion ($M_w/M_n$) of the obtained copolymer was measured using GPC, and as a result, it was found to be 1.53.

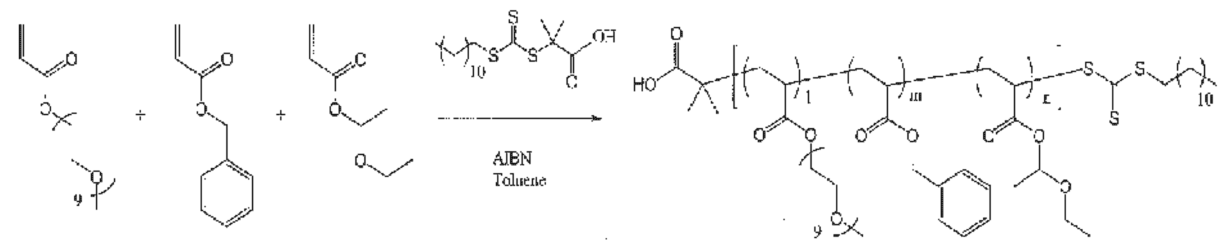


[Measurement apparatus and conditions]

(1) $^1$H-NMR measurement

Apparatus: JNM-ECX 400 (400 MHz)/JEOL Ltd.
Solvent: Dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Result: Fig. 1

(2) GPC measurement

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle diameter: 7 μm,
exclusion limit molecular weight: 5 × $10^3$)
TSKgel α-4000 column/Tosoh Corporation
(Column size: 7.8 mm × 00 mm, particle diameter: 10 μm,
exclusion limit molecular weight: 4 × $10^5$)
TSKgel guardcolum/Tosoh Corporation
Mobile phase: N,N-dimethyformamide (DMF) containing 10 mmol/L lithium bromide
Temperature: 40°C
Flow rate: 0.5 mL/min
Sample concentration: 6 mg/mL
Standard substance: Poly(methyl methacrylate) standard ReadyCal set, $M_p$ 800-2 200 000 Da/SIGMA
Result: Fig. 2

[Table 1]

| Example | Composition ratio (molar ratio to chain transfer agent) | | | | | Solvent | Temperature (°C) | Polymerization time (min) | Yield (g) | Polymerization degree | | | $M_{n,NMR}$ | $M_w/M_n$ |
|---|---|---|---|---|---|---|---|---|---|---|---|---|---|---|
| | Chain transfer agent | Initiator | Monomer | | | | | | | | | | | |
| | DDMAT | AIBN | mPEGA n=9 | BnA | EEA | | | | | mPEGA n=9 | BnA | EEA | | |
| 1 | 1 | 0.5 | 100 | 90 | 10 | Toluene | 70 | 90 | 1.223 | 102 | 94 | 9 | 65 900 | 1.53 |

[Examples 2 to 68]

Polymers having different composition ratios and average molecular weights shown in the following table were produced by appropriately changing the type, charged amount, reaction temperature, and polymerization time of the monomers (mPEGA, BnA, and EEA) used in Example 1, and using the same method as in Example 1.

[Table 2]

| Example | Composition ratio (molar ratio to chain transfer agent) | | | | | | | | | | | Solvent | Temperature (°C) | Polymerization time (min) |
|---|---|---|---|---|---|---|---|---|---|---|---|---|---|---|
| | Chain transfer agent | Initiator | | Monomer | | | | | | | | | | |
| | DDMAT | AIBN | ACHN | mPEGA | | | BnA | EEA | 2-Hydroxy ethyl acrylate | 4-Hydroxy butyl acrylate | 2-Hydroxy-3-phenoxy propyl acrylate | | | |
| | | | | n=4 | n=9 | n=22 | | | | | | | | |
| 2 | 1 | 2 | - | 255 | - | - | 15 | 30 | - | - | - | Toluene | 70 | 90 |
| 3 | 1 | 2 | - | 160 | - | - | 20 | 20 | - | - | - | Toluene | 70 | 60 |
| 4 | 1 | 2 | - | 240 | - | - | 30 | 30 | - | - | - | Toluene | 70 | 60 |
| 5 | 1 | 2 | - | 170 | - | - | 10 | 20 | - | - | - | Toluene | 70 | 60 |
| 6 | 1 | 2 | - | 255 | - | - | 15 | 30 | - | - | - | Toluene | 70 | 60 |
| 7 | 1 | 0.5 | - | - | 16 | - | 16 | 8 | - | - | - | Toluene | 70 | 90 |
| 8 | 1 | 0.5 | - | - | 18 | - | 14 | 8 | - | - | - | Toluene | 70 | 90 |
| 9 | 1 | 0.5 | - | - | 20 | - | 12 | 8 | - | - | - | Toluene | 70 | 90 |
| 10 | 1 | 0.5 | - | - | 20 | - | 12 | 8 | - | - | - | Toluene | 70 | 90 |
| 11 | 1 | 0.5 | - | - | 20 | - | 12 | 8 | - | - | - | Toluene | 70 | 90 |
| 12 | 1 | 0.5 | - | - | 20 | - | 16 | 4 | - | - | - | Toluene | 70 | 10 |
| 13 | 1 | 0.5 | - | - | 20 | - | 16 | 4 | - | - | - | Toluene | 70 | 30 |
| 14 | 1 | 0.5 | - | - | 20 | - | 16 | 4 | - | - | - | Toluene | 70 | 50 |
| 15 | 1 | 0.5 | - | - | 20 | - | 16 | 4 | - | - | - | Toluene | 70 | 70 |
| 16 | 1 | 0.5 | - | - | 20 | - | 16 | 4 | - | - | - | Toluene | 70 | 90 |
| 17 | 1 | 0.5 | - | - | 22 | - | 10 | 8 | - | - | - | Toluene | 70 | 90 |
| 18 | 1 | 0.5 | - | - | 24 | - | 8 | 8 | - | - | - | Toluene | 70 | 90 |
| 19 | 1 | 0.5 | - | - | 24 | - | 8 | 8 | - | - | - | Toluene | 70 | 90 |
| 20 | 1 | 0.5 | - | - | 28 | - | 4 | 8 | - | - | - | Toluene | 70 | 90 |
| 21 | 1 | 0.5 | - | - | 10 | - | 25 | 15 | - | - | - | Toluene | 70 | 90 |
| 22 | 1 | 0.5 | - | - | 17 | - | 25 | 8 | - | - | - | Toluene | 70 | 90 |
| 23 | 1 | 0.5 | - | - | 22.5 | - | 12.5 | 15 | - | - | - | Toluene | 70 | 90 |
| 24 | 1 | 0.5 | - | - | 25 | - | 20 | 5 | - | - | - | Toluene | 70 | 90 |
| 25 | 1 | 0.5 | - | - | 29.5 | - | 12.5 | 8 | - | - | - | Toluene | 70 | 90 |
| 26 | 1 | 0.5 | - | - | 30 | - | 24 | 6 | - | - | - | Toluene | 70 | 90 |
| 27 | 1 | 0.5 | - | - | 35 | - | 28 | 7 | - | - | - | Toluene | 70 | 90 |
| 28 | 1 | 0.5 | - | - | 50 | - | 40 | 10 | - | - | - | Toluene | 70 | 90 |
| 29 | 1 | 0.5 | - | - | 10 | - | 10 | 180 | - | - | - | Toluene | 70 | 90 |
| 30 | 1 | 0.5 | - | - | 30 | - | 30 | 140 | - | - | - | Toluene | 70 | 90 |
| 31 | 1 | 0.5 | - | - | 30 | - | 70 | 100 | - | - | - | Toluene | 70 | 90 |
| 32 | 1 | 0.5 | - | - | 30 | - | 110 | 60 | - | - | - | Toluene | 70 | 90 |
| 33 | 1 | 0.5 | - | - | 50 | - | 10 | 140 | - | - | - | Toluene | 70 | 90 |
| 34 | 1 | 0.5 | - | - | 50 | - | 50 | 100 | - | - | - | Toluene | 70 | 90 |
| 35 | 1 | 0.5 | - | - | 50 | - | 90 | 60 | - | - | - | Toluene | 70 | 90 |
| 36 | 1 | 0.5 | - | - | 50 | - | 130 | 20 | - | - | - | Toluene | 70 | 90 |
| 37 | 1 | 0.5 | - | - | 70 | - | 30 | 100 | - | - | - | Toluene | 70 | 90 |
| 38 | 1 | 0.5 | - | - | 70 | - | 70 | 60 | - | - | - | Toluene | 70 | 90 |
| 39 | 1 | 0.5 | - | - | 70 | - | 110 | 20 | - | - | - | Toluene | 70 | 90 |

| | | | | | | | | | | | | | | |
|---|---|---|---|---|---|---|---|---|---|---|---|---|---|---|
| 40 | 1 | 0.5 | - | - | 80 | - | 80 | 40 | - | - | - | Toluene | 70 | 90 |
| 41 | 1 | 0.5 | - | - | 80 | - | 100 | 20 | - | - | - | Toluene | 70 | 90 |
| 42 | 1 | 0.5 | - | - | 90 | - | 10 | 100 | - | - | - | Toluene | 70 | 90 |
| 43 | 1 | 0.5 | - | - | 90 | - | 50 | 60 | - | - | - | Toluene | 70 | 90 |
| 44 | 1 | 0.5 | - | - | 90 | - | 90 | 20 | - | - | - | Toluene | 70 | 90 |
| 45 | 1 | 0.5 | - | - | 100 | - | 20 | 80 | - | - | - | Toluene | 70 | 90 |
| 46 | 1 | 0.5 | - | - | 100 | - | 40 | 60 | - | - | - | Toluene | 70 | 90 |
| 47 | 1 | 0.5 | - | - | 100 | - | 50 | 50 | - | - | - | Toluene | 70 | 90 |
| 48 | 1 | 0.5 | - | - | 100 | - | 60 | 40 | - | - | - | Toluene | 70 | 90 |
| 49 | 1 | 0.5 | - | - | 100 | - | 70 | 30 | - | - | - | Toluene | 70 | 90 |
| 50 | 1 | 0.5 | - | - | 100 | - | 80 | 20 | - | - | - | Toluene | 70 | 90 |
| 51 | 1 | 0.5 | - | - | 110 | - | 30 | 60 | - | - | - | Toluene | 70 | 90 |
| 52 | 1 | 0.5 | - | - | 110 | - | 70 | 20 | - | - | - | Toluene | 70 | 90 |
| 53 | 1 | 0.5 | - | - | 130 | - | 10 | 60 | - | - | - | Toluene | 70 | 90 |
| 54 | 1 | 0.5 | - | - | 130 | - | 50 | 20 | - | - | - | Toluene | 70 | 90 |
| 55 | 1 | 0.5 | - | - | 150 | - | 30 | 20 | - | - | - | Toluene | 70 | 90 |
| 56 | 1 | 0.5 | - | - | 170 | - | 10 | 20 | - | - | - | Toluene | 70 | 90 |
| 57 | 1 | 0.5 | - | - | 200 | - | 160 | 40 | - | - | - | Toluene | 70 | 90 |
| 58 | 1 | 0.5 | - | - | 300 | - | 240 | 60 | - | - | - | Toluene | 70 | 90 |
| 59 | 1 | 0.5 | - | - | 400 | - | 320 | 80 | - | - | - | Toluene | 70 | 90 |
| 60 | 1 | 2 | - | - | - | 105 | 155 | 40 | - | - | - | Toluene | 70 | 90 |
| 61 | 1 | 2 | - | - | - | 120 | 240 | 40 | - | - | - | Toluene | 70 | 90 |
| 62 | 1 | 2 | - | - | - | 140 | 210 | 50 | - | - | - | Toluene | 70 | 90 |
| 63 | 1 | 2 | - | - | - | 140 | 220 | 40 | - | - | - | Toluene | 70 | 90 |
| 64 | 1 | 0.1 | - | - | 100 | - | 80 | - | 20 | - | - | 1,4-dioxane | 70 | 90 |
| 65 | 1 | - | 0.5 | - | 100 | - | 50 | - | - | 50 | - | 1,4-dioxane | 70 | 180 |
| 66 | 1 | - | 0.5 | - | 100 | - | 80 | - | - | 20 | - | 1,4-dioxane | 70 | 180 |
| 67 | 1 | 0.1 | - | - | 130 | - | 65 | - | - | - | 65 | 1,4-dioxane | 70 | 90 |
| 68 | 1 | 0.1 | - | - | 130 | - | 104 | - | - | - | 26 | 1,4-dioxane | 70 | 90 |

[Table 3]

| Example | Polymerization degree | | | | | | | | $M_{n,NMR}$ | $M_w/M_n$ |
|---|---|---|---|---|---|---|---|---|---|---|
| | mPEGA | | | BnA | EEA | 2-Hydroxy ethyl acrylate | 4-Hydroxy butyl acrylate | 2-Hydroxy-3-phenoxy propyl acrylate | | |
| | n=4 | n=9 | n=22 | | | | | | | |
| 2 | 250 | - | - | 18 | 31 | - | - | - | 62 400 | 1.37 |
| 3 | 155 | - | - | 22 | 20 | - | - | - | 39 200 | 1.23 |
| 4 | 211 | - | - | 29 | 29 | - | - | - | 53 100 | 1.32 |
| 5 | 162 | - | - | 11 | 20 | - | - | - | 39 000 | 1.22 |
| 6 | 227 | - | - | 15 | 28 | - | - | - | 54 300 | 1.33 |
| 7 | - | 16 | - | 16 | 8 | - | - | - | 11 800 | 1.20 |

| | | | | | | | | | | |
|---|---|---|---|---|---|---|---|---|---|---|
| 8 | - | 18 | - | 14 | 8 | - | - | - | 12 400 | 1.20 |
| 9 | - | 13 | - | 8 | 5 | - | - | - | 8 600 | 1.46 |
| 10 | - | 20 | - | 13 | 8 | - | - | - | 13 400 | 1.30 |
| 11 | - | 18 | - | 11 | 7 | - | - | - | 11 700 | 1.33 |
| 12 | - | 8 | - | 7 | 1 | - | - | - | 5 300 | 1.51 |
| 13 | - | 16 | - | 13 | 3 | - | - | - | 10 800 | 1.30 |
| 14 | - | 19 | - | 15 | 3 | - | - | - | 12 200 | 1.28 |
| 15 | - | 20 | - | 16 | 4 | - | - | - | 13 300 | 1.39 |
| 16 | - | 24 | - | 19 | 4 | - | - | - | 15 700 | 1.29 |
| 17 | - | 21 | - | 10 | 8 | - | - | - | 13 200 | 1.20 |
| 18 | - | 13 | - | 5 | 4 | - | - | - | 8 000 | 1.44 |
| 19 | - | 24 | - | 8 | 8 | - | - | - | 14 300 | 1.21 |
| 20 | - | 26 | - | 4 | 8 | - | - | - | 14 600 | 1.20 |
| 21 | - | 7 | - | 17 | 9 | - | - | - | 8 100 | 1.41 |
| 22 | - | 11 | - | 17 | 5 | - | - | - | 9 200 | 1.39 |
| 23 | - | 15 | - | 9 | 10 | - | - | - | 10 700 | 1.30 |
| 24 | - | 26 | - | 21 | 5 | - | - | - | 16 900 | 1.31 |
| 25 | - | 20 | - | 10 | 6 | - | - | - | 12 400 | 1.33 |
| 26 | - | 33 | - | 27 | 6 | - | - | - | 21 500 | 1.34 |
| 27 | - | 39 | - | 30 | 7 | - | - | - | 25 000 | 1.36 |
| 28 | - | 39 | - | 4 | 8 | - | - | - | 20 200 | 1.25 |
| 29 | - | 10 | - | 10 | 159 | - | - | - | 29 700 | 1.19 |
| 30 | - | 27 | - | 27 | 122 | - | - | - | 35 600 | 1.27 |
| 31 | - | 30 | - | 66 | 91 | - | - | - | 38 600 | 1.17 |
| 32 | - | 27 | - | 91 | 52 | - | - | - | 35 800 | 1.20 |
| 33 | - | 45 | - | 10 | 123 | - | - | - | 41 000 | 1.31 |
| 34 | - | 44 | - | 45 | 86 | - | - | - | 41 100 | 1.20 |
| 35 | - | 45 | - | 79 | 54 | - | - | - | 42 700 | 1.24 |
| 36 | - | 43 | - | 111 | 19 | - | - | - | 42 000 | 1.28 |
| 37 | - | 60 | - | 28 | 87 | - | - | - | 46 500 | 1.24 |
| 38 | - | 55 | - | 57 | 49 | - | - | - | 43 000 | 1.27 |
| 39 | - | 56 | - | 89 | 18 | - | - | - | 44 300 | 1.30 |
| 40 | - | 79 | - | 80 | 36 | - | - | - | 56 400 | 1.43 |
| 41 | - | 81 | - | 106 | 17 | - | - | - | 58 900 | 1.51 |
| 42 | - | 81 | - | 12 | 93 | - | - | - | 54 600 | 1.27 |
| 43 | - | 71 | - | 42 | 51 | - | - | - | 48 600 | 1.30 |
| 44 | - | 70 | - | 73 | 18 | - | - | - | 48 400 | 1.32 |
| 45 | - | 84 | - | 19 | 72 | - | - | - | 54 200 | 1.33 |
| 46 | - | 80 | - | 35 | 52 | - | - | - | 51 900 | 1.35 |
| 47 | - | 84 | - | 45 | 44 | - | - | - | 54 200 | 1.33 |
| 48 | - | 82 | - | 52 | 35 | - | - | - | 53 100 | 1.36 |
| 49 | - | 94 | - | 68 | 26 | - | - | - | 60 100 | 1.50 |
| 50 | - | 104 | - | 83 | 20 | - | - | - | 66 700 | 1.52 |
| 51 | - | 93 | - | 30 | 55 | - | - | - | 57 700 | 1.33 |
| 52 | - | 84 | - | 56 | 18 | - | - | - | 52 400 | 1.35 |
| 53 | - | 105 | - | 10 | 52 | - | - | - | 60 000 | 1.35 |

| | | | | | | | | | | |
|---|---|---|---|---|---|---|---|---|---|---|
| 54 | - | 102 | - | 42 | 18 | - | - | - | 58 900 | 1.37 |
| 55 | - | 111 | - | 25 | 18 | - | - | - | 60 100 | 1.40 |
| 56 | - | 131 | - | 10 | 18 | - | - | - | 67 400 | 1.43 |
| 57 | - | 129 | - | 108 | 31 | - | - | - | 84 100 | 1.51 |
| 58 | - | 170 | - | 144 | 40 | - | - | - | 111 200 | 1.61 |
| 59 | - | 221 | - | 191 | 50 | - | - | - | 144 700 | 1.68 |
| 60 | - | - | 58 | 102 | 78 | - | - | - | 91 200 | 1.71 |
| 61 | - | - | 62 | 143 | 21 | - | - | - | 93 100 | 1.68 |
| 62 | - | - | 59 | 105 | 93 | - | - | - | 95 000 | 1.82 |
| 63 | - | - | 60 | 106 | 18 | - | - | - | 85 400 | 1.68 |
| 64 | - | 75 | - | 66 | - | 19 | - | - | 49 400 | 1.46 |
| 65 | - | 67 | - | 37 | - | - | 39 | - | 44 300 | 1.40 |
| 66 | - | 61 | - | 56 | - | - | 20 | - | 41 600 | 1.43 |
| 67 | - | 75 | - | 42 | - | - | - | 42 | 52 300 | 1.61 |
| 68 | - | 75 | - | 68 | - | - | - | 18 | 51 400 | 1.56 |

[Example 69]

110 mg of 2-(dodecylthiocarbonothioylthio)-2-methylpropionic acid (DDMAT) was weighed and dissolved in 19.0 mL of toluene to prepare a DDMAT/toluene stock solution (5.78 mg/mL as a DDMAT concentration). Similarly, 25 mg of 2,2'-azobis(2-methylpropionitrile) (AIBN) was weighed and dissolved in 19.7 mL of toluene to prepare an AIBN/toluene stock solution (AIBN concentration: 1.27 mg/mL). Separately, 12.96 g of poly(ethylene glycol) methyl ether acrylate (mPEGA, the average value (n) of the numbers of repetitions of ethylene glycol is 9), 3.50 g of benzyl acrylate (BnA), 0.78 g of 1-ethoxyethyl acrylate, 17.3 mL of a DDMAT/toluene stock solution, and 17.3 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally the vacuum drying was performed. By treating obtained copolymer with 0.5N HCl at room temperature, an ethoxyethyl group was eliminated to obtain 12.41 g of poly[(benzyl acrylate)-co-(poly(ethylene glycol)methyl ether acrylate)-co-(acrylic acid)].

As a result of analyzing a polymerization degree of each monomer and a number average molecular weight ($M_{n,NMR}$) of the obtained copolymer from a $^1$H-NMR spectrum measured by using NMR, the polymerization degree of mPEGA (n = 9) was 88, the polymerization degree of BnA was 75, the polymerization degree of EEA was 17, and $M_{n,NMR}$ was 57 200. Moreover, a molecular weight dispersion ($M_w/M_n$) of the obtained copolymer was measured by using the GPC and a result thereof was 1.51.

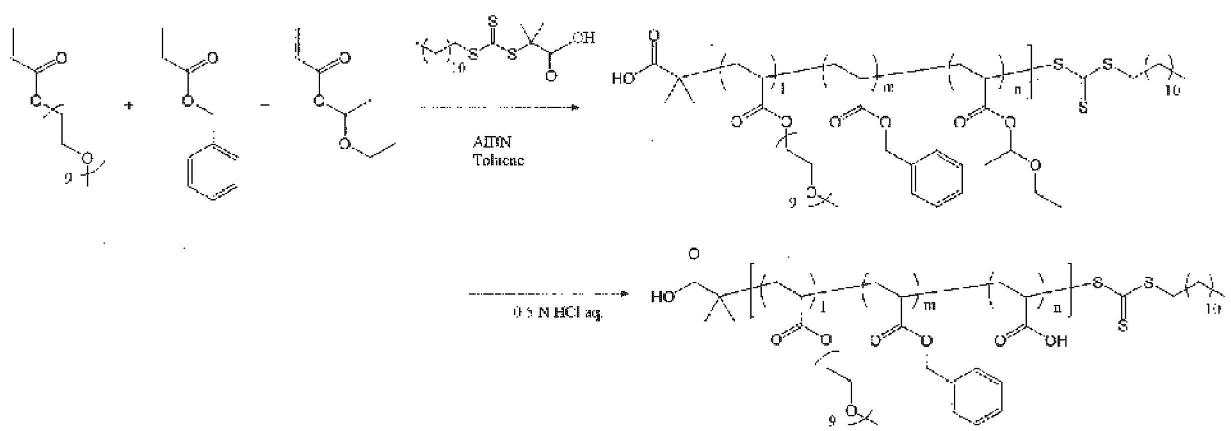


[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/ KANTO CHEMICAL CO., INC.
Sample concentration: 20 mg/mL
Measurement temperature: 25°C
Number of integration times: 256 times
Results: Fig. 3

(2) GPC measurement

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle diameter: 7 μm, and exclusion limit molecular weight: 5 × $10^3$)
TSKgel α-4000 column/Tosoh Corporation
(Column size: 7.8 mm × 300 mm, particle diameter: 10 μm, and exclusion limit molecular weight: 4 × $10^5$)
TSKgel guardcolum/Tosoh Corporation
Mobile phase: N,N-dimethyformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, $M_p$ 800 - 2 200 000 Da/SIGMA
Results: Fig. 4

[Table 4]

| Example | Composition ratio (molar ratio to chain transfer agent) | | | | | Solvent | Temperature (°C) | Polymerization time | Yield (g) | Polymerization degree | | | $M_{n,NMR}$ | $M_w/M_n$ |
|---|---|---|---|---|---|---|---|---|---|---|---|---|---|---|
| | Chain transfer agent | Initiator | Monomer | | | | | | | | | | | |
| | DDMAT | AIBN | mPEGA n=9 | BnA | EEA | | | | | mPEGA n=9 | BnA | EEA | | |
| 69 | 1 | 0.5 | 100 | 80 | 20 | Toluene | 70 | 90 | 12.41 | 88 | 75 | 17 | 57 200 | 1.51 |

[Examples 70 to 97]

Polymers having different composition ratios and average molecular weights as shown in the following table were produced by appropriately changing charged amounts and polymerization times of the monomers (mPEGA, BnA, and EEA) used in Example 69, by the same method as Example 69.

[Table 5]

| Example | Composition ratio (molar ratio to chain transfer agent) | | | | | Solvent | Temperature (°C) | Polymerization time (min) |
|---|---|---|---|---|---|---|---|---|
| | Chain transfer agent | Initiator | Monomer | | | | | |
| | DDMAT | AIBN | mPEGA (n=9) | BnA | EEA | | | |
| 70 | 1 | 0.5 | 16 | 10 | 6 | Toluene | 70 | 30 |
| 71 | 1 | 0.5 | 10 | 25 | 15 | Toluene | 70 | 95 |
| 72 | 1 | 0.5 | 17 | 25 | 8 | Toluene | 70 | 95 |
| 73 | 1 | 0.5 | 22.5 | 12.5 | 15 | Toluene | 70 | 95 |
| 74 | 1 | 0.5 | 25 | 10 | 15 | Toluene | 70 | 60 |
| 75 | 1 | 0.5 | 25 | 12.5 | 12.5 | Toluene | 70 | 120 |
| 76 | 1 | 0.5 | 29.5 | 12.5 | 8 | Toluene | 70 | 95 |
| 77 | 1 | 0.5 | 32.5 | 13 | 19.5 | Toluene | 70 | 100 |
| 78 | 1 | 0.5 | 40 | 16 | 24 | Toluene | 70 | 100 |
| 79 | 1 | 0.5 | 40 | 20 | 20 | Toluene | 70 | 100 |
| 80 | 1 | 0.5 | 50 | 20 | 30 | Toluene | 70 | 100 |
| 81 | 1 | 0.5 | 50 | 25 | 25 | Toluene | 70 | 100 |
| 82 | 1 | 0.5 | 75 | 30 | 45 | Toluene | 70 | 100 |
| 83 | 1 | 0.5 | 75 | 37.5 | 37.5 | Toluene | 70 | 100 |
| 84 | 1 | 0.5 | 50 | 10 | 140 | Toluene | 70 | 90 |
| 85 | 1 | 0.5 | 50 | 50 | 100 | Toluene | 70 | 90 |
| 86 | 1 | 0.5 | 50 | 90 | 60 | Toluene | 70 | 90 |
| 87 | 1 | 0.5 | 70 | 70 | 60 | Toluene | 70 | 90 |
| 88 | 1 | 0.5 | 70 | 110 | 20 | Toluene | 70 | 90 |
| 89 | 1 | 0.5 | 80 | 100 | 20 | Toluene | 70 | 90 |
| 90 | 1 | 0.5 | 90 | 90 | 20 | Toluene | 70 | 90 |
| 91 | 1 | 0.5 | 100 | 20 | 80 | Toluene | 70 | 90 |
| 92 | 1 | 0.5 | 100 | 40 | 60 | Toluene | 70 | 90 |
| 93 | 1 | 0.5 | 100 | 50 | 50 | Toluene | 70 | 90 |
| 94 | 1 | 0.5 | 100 | 60 | 40 | Toluene | 70 | 90 |
| 95 | 1 | 0.5 | 100 | 90 | 10 | Toluene | 70 | 90 |
| 96 | 1 | 0.5 | 200 | 160 | 40 | Toluene | 70 | 90 |
| 97 | 1 | 0.5 | 400 | 320 | 80 | Toluene | 70 | 90 |

[Table 6]

| Example | Polymerization degree | | | $M_{n,NMR}$ | $M_w/M_n$ |
|---|---|---|---|---|---|
| | Monomer | | | | |
| | mPEGA (n=9) | BnA | EEA (Acrylic acid) | | |
| 70 | 13 | 8 | 5 | 8 200 | 1.29 |
| 71 | 7 | 17 | 9 | 7 400 | 1.41 |
| 72 | 11 | 17 | 5 | 8 800 | 1.39 |
| 73 | 15 | 9 | 10 | 10 000 | 1.30 |
| 74 | 18 | 8 | 11 | 11 000 | 1.34 |
| 75 | 19 | 11 | 10 | 11 900 | 1.37 |
| 76 | 20 | 10 | 6 | 12 000 | 1.33 |
| 77 | 33 | 14 | 18 | 19 700 | 1.13 |
| 78 | 40 | 18 | 25 | 24 100 | 1.14 |
| 79 | 39 | 22 | 21 | 24 300 | 1.14 |

(continued)

| Example | Polymerization degree | | | $M_{n,NMR}$ | $M_w/M_n$ |
|---|---|---|---|---|---|
| | Monomer | | | | |
| | mPEGA (n=9) | BnA | EEA (Acrylic acid) | | |
| 80 | 48 | 21 | 30 | 28 800 | 1.16 |
| 81 | 49 | 27 | 27 | 30 100 | 1.16 |
| 82 | 67 | 29 | 42 | 40 100 | 1.21 |
| 83 | 69 | 37 | 37 | 42 000 | 1.22 |
| 84 | 45 | 10 | 123 | 32 200 | 1.31 |
| 85 | 44 | 45 | 86 | 34 900 | 1.20 |
| 86 | 45 | 79 | 54 | 38 800 | 1.24 |
| 87 | 55 | 57 | 49 | 39 400 | 1.27 |
| 88 | 56 | 89 | 18 | 43 000 | 1.30 |
| 89 | 81 | 106 | 17 | 57 700 | 1.51 |
| 90 | 70 | 73 | 18 | 47 200 | 1.32 |
| 91 | 84 | 19 | 72 | 49 000 | 1.32 |
| 92 | 80 | 35 | 52 | 48 100 | 1.34 |
| 93 | 84 | 45 | 44 | 51 000 | 1.33 |
| 94 | 82 | 52 | 35 | 50 600 | 1.36 |
| 95 | 102 | 94 | 9 | 65 200 | 158 |
| 96 | 129 | 108 | 31 | 81 900 | 1.51 |
| 97 | 221 | 191 | 50 | 141 100 | 1.68 |

[Example 98]

End structure conversion of terpolymer

In toluene (70 mL), the copolymer obtained in Example 69 (7.00 g) was dissolved. To this solution, AIBN (411 mg) and lauroyl peroxide (100 mg) were added, and the mixture was stirred in a hot water bath at 80°C overnight. After the reaction was stopped by ice cooling, the copolymer was recovered by subjecting the reaction solution to reprecipitation method or dialyzed against methanol. Since the obtained copolymer was basically a viscous body, in the reprecipitation, an operation of dropping the reaction solution into a centrifuge tube to which the poor solvent (hexane/ethyl acetate = 7/3 [v/v]) was added and recovering the solution by centrifugation (2 000 × g, 5 min) was repeated three times, and finally the vacuum drying was performed to obtain a terpolymer with a converted end structure (6.25 g).
A residual ratio of the end structure of the obtained copolymer was evaluated based on an absorbance of a peak derived from a trithiocarbonate group (wavelength: 309 nm) in a UV spectrum measured by using an ultraviolet-visible spectrophotometer, and a result thereof was 0.0%.

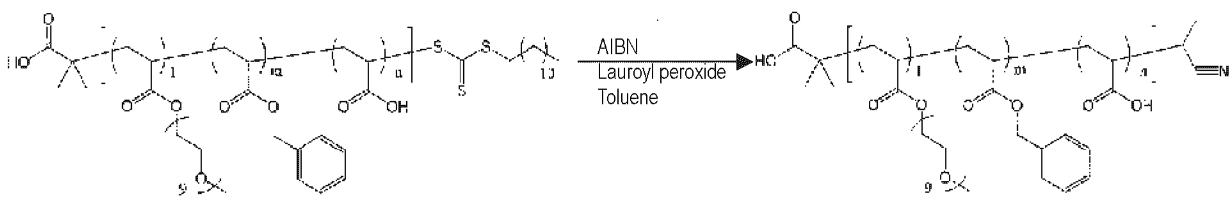


[Measurement apparatuses and conditions]

(1) UV spectrum measurement

Apparatus: Hitachi spectrophotometer U-9300/Hitachi, Ltd.
Solvent: purified water
Sample concentration: 4 mg/mL
Measurement wavelength: 250 to 500 nm
Results: Fig. 5

[Table 7]

| Example | Copolymer used | Azo compound used in reaction | Residual ratio of end structure (%) |
|---|---|---|---|
| 98 | Example 69 | AIBN | 0.0 |

[Examples 99 to 105]

Copolymers having different end structures as shown in the following table were synthesized by appropriately changing a type and a charged amount of the azo compound for the copolymers obtained in Examples 70 to 97 by the same method as Example 98.

[Table 8]

| Example | Copolymer used | Azo compound used in reaction | Residual ratio of end structure (%) |
|---|---|---|---|
| 99 | Example 70 | AIBN | 0.0 |
| 100 | Example 74 | AIBN | 0.0 |
| 101 | Example 82 | AIBN | 0.0 |
| 102 | Example 92 | AIBN | 3.6 |
| 103 | Example 93 | AIBN | 0.0 |
| 104 | Example 82 | MAIB | 0.0 |
| 105 | Example 93 | MAIB | 0.0 |

[Example 106]

Synthesis of chelating agent-bonded copolymer (disulfide bond)

In DMF (13 mL), the copolymer obtained in Example 98 (650 mg) was dissolved, (1-cyano-2-ethoxy-2-oxoethylideneaminooxy)dimethylaminomorpholinocarbenium hexafluorophosphate (COMU) (166 mg) and 2,2,6,6-tetramethylpiperidine (TMP) (66 μL) were added, and the mixture was stirred at room temperature for 3 hours. Thereto was added Boc-cystamine hydrochloride (280 mg), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of dichloromethane (DCM) and trifluoroacetic acid (TFA) [DCM/TFA = 5/3 (v/v)] (10 mL), and the mixture was stirred at room temperature overnight to perform deprotection. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. In DMF (21 mL), the obtained copolymer (534 mg) was dissolved, DOTA-tris(t-Bu ester) (387 mg), COMU (289 mg), and TMP (114 μL) were added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (32 mL) and the mixture was stirred at room temperature overnight to perform carboxyl group deprotection, and then the solvent was removed by

the distillation under reduced pressure and the vacuum drying to obtain the chelating agent-bonded copolymer (disulfide linker) (557 mg).

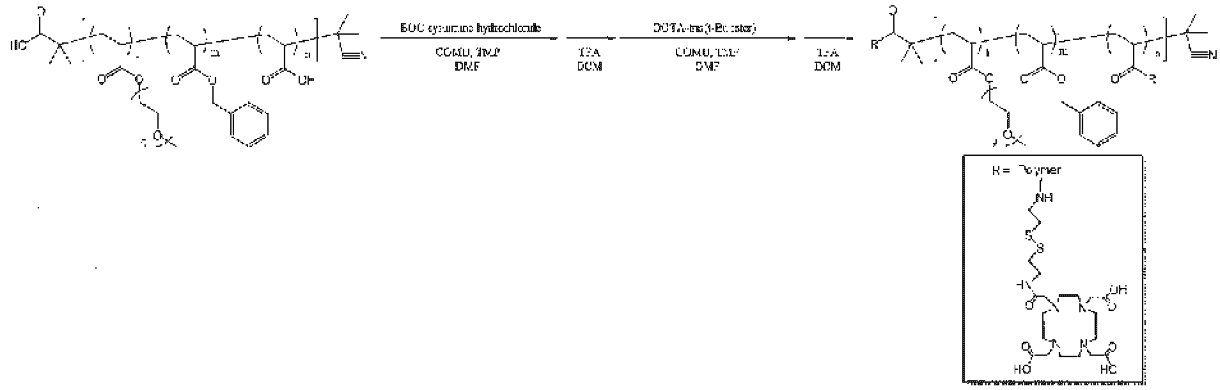


For the chelating agent-bonded copolymer (disulfide linker) before the carboxyl group deprotection, the number of the chelating agent introduced to one molecule of the copolymer was analyzed from a $^1$H-NMR spectrum measured by using NMR, and a result thereof was 11 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 6

[Table 9]

| Example | Copolymer used | Number of chelating agent introduced to one molecule of copolymer (mol/mol) |
|---|---|---|
| 106 | Example 98 | 11 |

[Examples 107 to 133]

For the copolymers obtained in Examples 69 to 105, copolymers having different numbers of the chelating agent introduced to one molecule of each copolymer as shown in the following table were synthesized by appropriately changing charged amounts of the linker and the chelating agent by the same method as Example 106.

[Table 10]

| Example | Copolymer used | Number of chelating agent introduced to one molecule of copolymer (mol/mol) |
|---|---|---|
| 107 | Example 69 | 11 |
| 108 | Example 70 | 2 |
| 109 | Example 74 | 4 |
| 110 | Example 75 | 4 |
| 111 | Example 78 | 10 |
| 112 | Example 79 | 8 |

(continued)

| Example | Copolymer used | Number of chelating agent introduced to one molecule of copolymer (mol/mol) |
|---|---|---|
| 113 | Example 80 | 13 |
| 114 | Example 81 | 12 |
| 115 | Example 82 | 19 |
| 116 | Example 83 | 18 |
| 117 | Example 84 | 28 |
| 118 | Example 85 | 29 |
| 119 | Example 86 | 22 |
| 120 | Example 87 | 20 |
| 121 | Example 88 | 10 |
| 122 | Example 90 | 10 |
| 123 | Example 91 | 30 |
| 124 | Example 92 | 23 |
| 125 | Example 93 | 25 |
| 126 | Example 94 | 19 |
| 127 | Example 99 | 3 |
| 128 | Example 100 | 5 |
| 129 | Example 101 | 10 |
| 130 | Example 102 | 28 |
| 131 | Example 103 | 25 |
| 132 | Example 104 | 12 |
| 133 | Example 105 | 18 |

[Example 134]

Synthesis of chelating agent-bonded copolymer (amide bond 1)

In DMF (16 mL), the copolymer obtained in Example 69 (400 mg) was dissolved, COMU (153 mg) and TMP (61 μL) were added, and the mixture was stirred at room temperature for 3 hours. Then, N-(tert-butoxycarbonyl)-1,2-diaminoethane (58 mg) was added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (16 mL), and the mixture was stirred at room temperature overnight to perform deprotection. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. In DMF (28 mL), the obtained copolymer (276 mg) was dissolved, DOTA-tris(t-Bu ester) (96 mg), COMU (89 mg), and TMP (70 μL) were added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (16 mL) and the mixture was stirred at room temperature overnight to perform carboxyl group deprotection, and then the solvent was removed by the distillation under reduced pressure and the vacuum drying to obtain the chelating agent-bonded copolymer (amide bond 1) (242 mg).

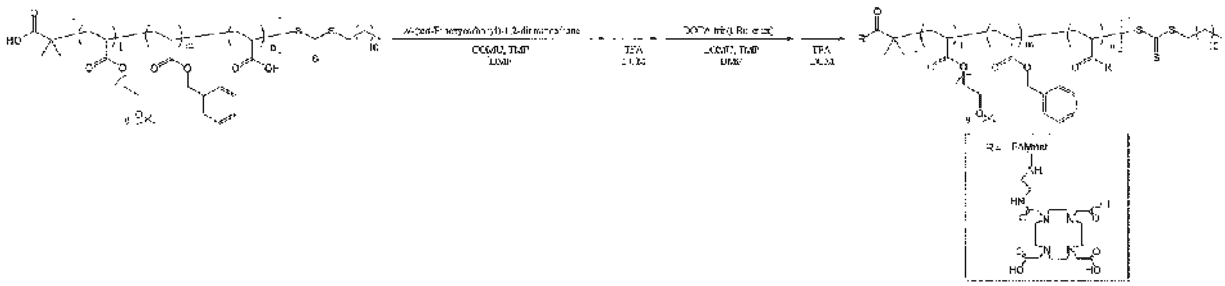

For the chelating agent-bonded copolymer (amide bond 1) before the carboxyl group deprotection, the number of the chelating agent introduced to one molecule of the copolymer was analyzed from a $^1$H-NMR spectrum measured by using NMR, and a result thereof was 11 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 7

[Table 11]

| Example | Copolymer used | Number of chelating agent introduced to one molecule of copolymer (mol/mol) |
|---|---|---|
| 134 | Example 69 | 11 |

[Examples 135 to 141]

For the copolymers obtained in Examples 71 to 78, copolymers having different numbers of the chelating agent introduced to one molecule of each copolymer as shown in the following table were synthesized by appropriately changing charged amounts of the linker and the chelating agent by the same method as Example 134.

[Table 12]

| Example | Copolymer used | Number of chelating agent introduced to one molecule of copolymer (mol/mol) |
|---|---|---|
| 135 | Example 71 | 8 |
| 136 | Example 72 | 4 |
| 137 | Example 73 | 8 |
| 138 | Example 75 | 5 |
| 139 | Example 76 | 5 |
| 140 | Example 77 | 9 |
| 141 | Example 78 | 10 |

[Example 142]

Synthesis of chelating agent-bonded copolymer (amide bond 2)

To a solution of the copolymer obtained in Example 98 (300 mg) in DMF (15 mL), were added COMU (65 mg) and TMP (26 uL), and the mixture was stirred at room temperature for 3 hours. Thereto was added p-$NH_2$-Bn-DOTA-tetra(t-Bu ester) (114 mg), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (32 mL) and the mixture was stirred at room temperature overnight to perform carboxyl group deprotection, and then the solvent was removed by the distillation under reduced pressure and the vacuum drying to obtain the chelating agent-bonded copolymer (amide bond 2) (281 mg).

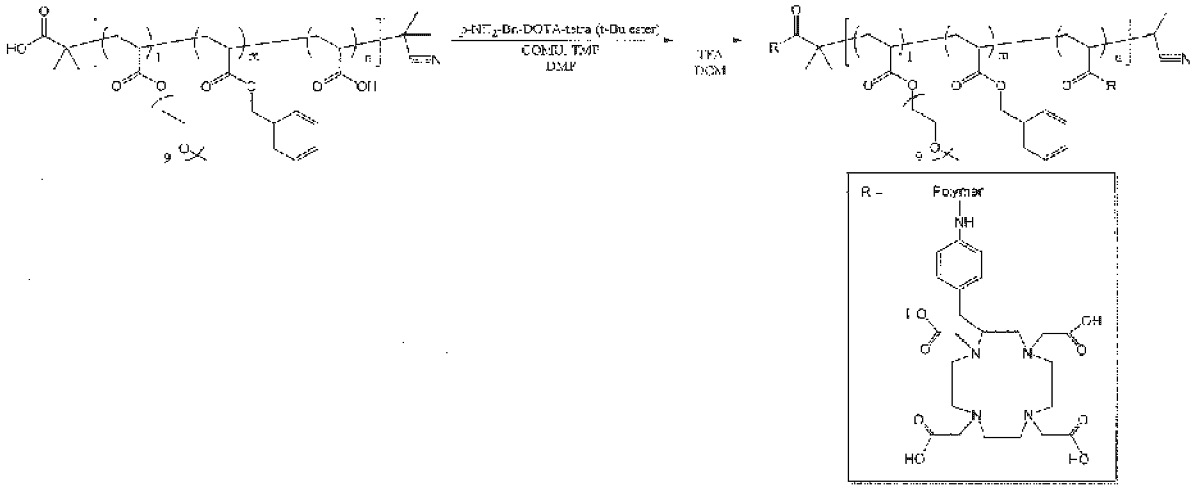


For the chelating agent-bonded copolymer (amide bond 2) before the carboxyl group deprotection, the number of the chelating agent introduced to one molecule of the copolymer was analyzed from a $^{1}$H-NMR spectrum measured by using NMR, and a result thereof was 7 mol/mol.

[Measurement apparatuses and conditions]

(1) $^{1}$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 8

[Table 13]

| Example | Copolymer used | Number of chelating agent introduced to one molecule of copolymer (mol/mol) |
|---|---|---|
| 142 | Example 98 | 7 |

[Examples 143 to 149]

For the copolymers obtained in Examples 69 to 97, copolymers having different numbers of the chelating agent introduced to one molecule of each copolymer as shown in the following table were synthesized by appropriately changing charged amounts of the linker and the chelating agent by the same method as Example 142.

[Table 14]

| Example | Copolymer used | Number of chelating agent introduced to one molecule of copolymer (mol/mol) |
|---|---|---|
| 143 | Example 69 | 18 |
| 144 | Example 89 | 15 |
| 145 | Example 90 | 14 |
| 146 | Example 93 | 30 |
| 147 | Example 95 | 9 |
| 148 | Example 96 | 16 |
| 149 | Example 97 | 20 |

[Example 150]

Synthesis of chelating agent-bonded copolymer (ester bond 1)

In dichloromethane (7 mL), the copolymer obtained in Example 98 (400 mg) was dissolved, 1-(3-dimethylaminopropyl)-3-ethylcarbodiimide hydrochloride (WSC-HCl) (69 mg) and 4-(dimethylamino)pyridine (DMAP) (44 mg) were added, and the mixture was stirred at room temperature for 3 hours. Then, 2-(tert-butoxycarbonylamino)-1-ethanol (183 μL) was added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in the solution mixture of DCM and TFA [DCM/TFA = 5/3 (v/v)] (16 mL), and the mixture was stirred at room temperature overnight to perform deprotection. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. In DMF (7 mL), the obtained copolymer (350 mg) was dissolved, DOTA-tris(t-Bu ester) (118 mg), COMU (88 mg), and TMP (35 μL) were added, and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (16 mL) and the mixture was stirred at room temperature overnight to perform carboxyl group deprotection, and then the solvent was removed by the distillation under reduced pressure and the vacuum drying to obtain the chelating agent-bonded copolymer (ester bond 1) (238 mg).

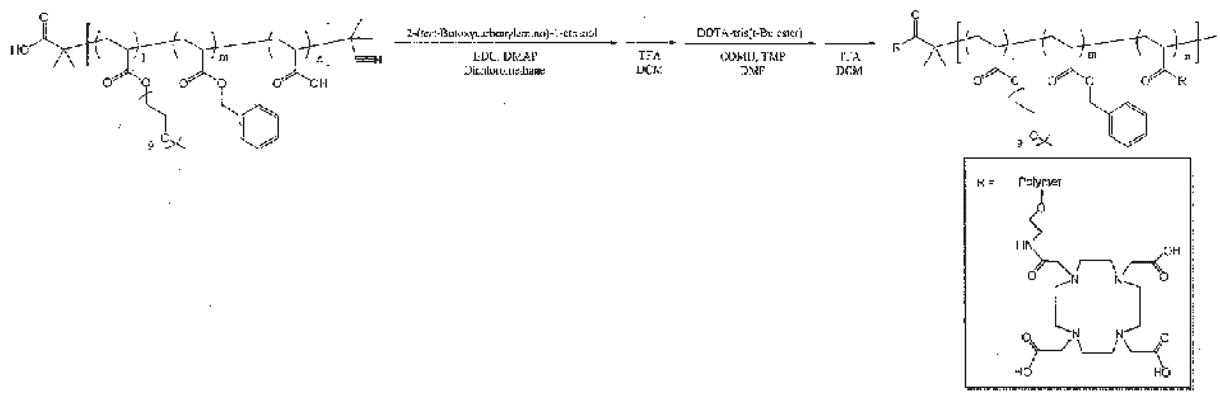


For the chelating agent-bonded copolymer (ester bond 1) before the carboxyl group deprotection, the number of the chelating agent introduced to one molecule of the copolymer was analyzed from a $^1$H-NMR spectrum measured by using NMR, and a result thereof was 12 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 9

[Table 15]

| Example | Copolymer used | Number of chelating agent introduced to one molecule of copolymer (mol/mol) |
|---|---|---|
| 150 | Example 98 | 12 |

[Example 151]

Synthesis of chelating agent-bonded copolymer (ester bond 2)

To a solution of the copolymer obtained in Example 100 (400 mg) in dichloromethane (7 mL), were added WSC-HCl (69 mg) and DMAP (44 mg), and the mixture was stirred at room temperature for 3 hours. Thereto was added tert-butyl glycolate (111 μL), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (16 mL), and the mixture was stirred at room temperature overnight to perform carboxyl group deprotection. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. To a solution of the obtained copolymer (350 mg) in DMF (7 mL), were added p-$NH_2$-Bn-DOTA-tetra (t-Bu ester) (174 mg), COMU (88 mg), and TMP (35 μL), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. The obtained copolymer was dissolved in a solution mixture of DCM and TFA [DCM/TFA = 5/3 (v/v)] (16 mL) and the mixture was stirred at room temperature overnight to perform carboxyl group deprotection, and then the solvent was removed by the distillation under reduced pressure and the vacuum drying to obtain the chelating agent-bonded copolymer (ester bond 2) (54 mg).

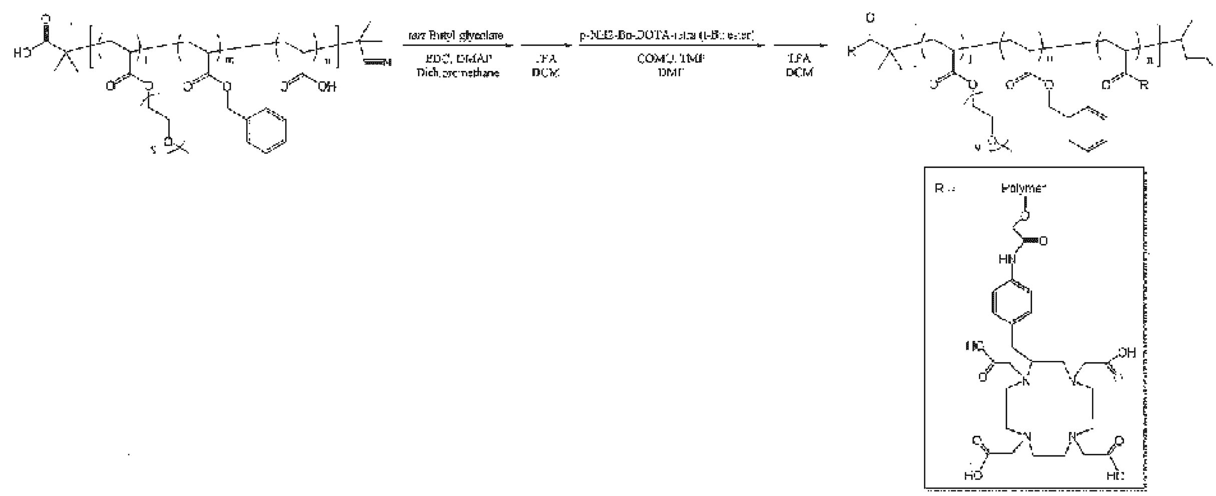


For the chelating agent-bonded copolymer (ester bond 2) before the carboxyl group deprotection, the number of

the chelating agent introduced to one molecule of the copolymer was analyzed from a $^1$H-NMR spectrum measured by using NMR, and a result thereof was 13 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 10

[Table 16]

| Example | Copolymer used | Number of chelating agent introduced to one molecule of copolymer (mol/mol) |
|---|---|---|
| 151 | Example 98 | 13 |

[Example 152]

Preparation of Gd-DOTA-bonded SCNP

In purified water (50 mL), the chelating agent-bonded copolymer obtained in Example 106 (500 mg) was dissolved and the mixture was bubbled with argon for 20 minutes. After addition of gadolinium chloride hexahydrate (313 mg), the solution was adjusted to pH 6.5 with 1N aqueous sodium hydroxide solution. This solution was stirred at 60°C for 3 hours to perform a complex formation reaction between Gd and DOTA. Then, dialysis purification was performed using purified water as an external liquid (dialysis membrane: Spectra/Por Regenerated Cellulose Membrane 6, molecular cut off: 3.5 kDa, external liquid: purified water) to roughly purify free Gd. The free Gd was precipitated as phosphate by adding 10-fold concentrated Dulbecco's phosphate buffered saline (D-PBS(-)) (8.5 mL) and stirring for 30 minutes. This solution was filtered through a filter (pore diameter: 0.2 μm), and then dialyzed against purified water again to remove salts contained in the buffer. An obtained inner fluid of the dialysis membrane was lyophilized to obtain the Gd-DOTA-bonded SCNP (356 mg).

The number of Gd bonded to one molecule of the copolymer of the Gd-DOTA-bonded SCNP obtained after the purification was measured by inductively coupled plasma-atomic emission spectrometry (ICP-AES), and was 9.4 mol/mol. Further, a Z-average particle diameter and a polydispersity index of the Gd-DOTA-bonded SCNP when dispersed in PBS were measured by dynamic light scattering (DLS), and a result of the Z-average particle diameter was 9.3 nm (polydispersity index: 0.15).

[Measurement apparatuses and conditions]

(1) ICP-AES measurement

Apparatus: sequential high frequency plasma light emitting apparatus ICPE-9000/SHIMADZU CORPORATION
Pretreatment apparatus: microwave sample pretreatment apparatus ETHOS EASY/Milestone General
Measurement wavelength: 342 nm
Standard solution: gadolinium standard solution (Gd1000) for ICP analysis/FUJIFILM Wako Pure Chemical Corporation
Internal standard substance: yttrium standard solution (Y1000) for ICP analysis/FUJIFILM Wako Pure Chemical Corporation
Sample concentration: 10 mg/mL (in terms of polymer)

(2) DLS measurement

Apparatus: Zetasizer NanoZS/Malvern Instruments Ltd.

Measurement temperature: 25°C

Sample concentration: 10 mg/mL

Results: Fig. 11

[Table 17]

| Example | Copolymer used | Number of Gd bonded to one molecule of copolymer (mol/mol) | Z-average particle diameter (nm) | Polydispersity index |
|---|---|---|---|---|
| 152 | Example 106 | 3.4 | 9.3 | 0.15 |

[Examples 153 to 198]

For the chelating agent-bonded copolymers obtained in Examples 107 to 151, contrast agent molecule-bonded SCNPs having different numbers of the paramagnetic metal bonded to one molecule of each copolymer as shown in the following table were synthesized by appropriately changing a type and a charged amount of the paramagnetic metal, and using a similar method as in Example 152.

[Table 18]

| Example | Copolymer used | Paramagnetic metal element | Number of paramagnetic metal element bonded to one molecule of copolymer (mol/mol) | Z-average particle diameter (nm) | Polydispersity index |
|---|---|---|---|---|---|
| 153 | Example 107 | Gd | 9.5 | 8.3 | 0.14 |
| 154 | Example 108 | Gd | 0.8 | 8.1 | 0.28 |
| 155 | Example 109 | Gd | 3.2 | 6.2 | 0.28 |
| 156 | Example 110 | Gd | 2.9 | 6.5 | 0.26 |
| 157 | Example 111 | Gd | 8.4 | 6.6 | 0.17 |
| 158 | Example 112 | Gd | 7.7 | 6.4 | 0.12 |
| 159 | Example 113 | Gd | 10.7 | 7.0 | 0.15 |
| 160 | Example 114 | Gd | 11.2 | 7.1 | 0.17 |
| 161 | Example 115 | Gd | 17.3 | 8.7 | 0.16 |
| 162 | Example 116 | Gd | 15.9 | 8.6 | 0.18 |
| 163 | Example 117 | Gd | 26.1 | 17.1 | 0.39 |
| 164 | Example 118 | Gd | 18.1 | 19.0 | 0.41 |
| 165 | Example 119 | Gd | 14.9 | 10.4 | 0.20 |
| 166 | Example 120 | Gd | 16.5 | 11.1 | 0.30 |
| 167 | Example 121 | Gd | 8.1 | 9.9 | 0.12 |
| 168 | Example 122 | Gd | 6.3 | 10.2 | 0.15 |
| 169 | Example 123 | Gd | 35.1 | 10.3 | 0.17 |
| 170 | Example 124 | Gd | 19.6 | 10.9 | 0.21 |

(continued)

| Example | Copolymer used | Paramagnetic metal element | Number of paramagnetic metal element bonded to one molecule of copolymer (mol/mol) | Z-average particle diameter (nm) | Polydispersity index |
|---|---|---|---|---|---|
| 171 | Example 125 | Gd | 22.7 | 10.0 | 0.21 |
| 172 | Example 126 | Gd | 17.6 | 10.9 | 0.27 |
| 173 | Example 127 | Gd | 2.2 | 10.2 | 0.58 |
| 174 | Example 128 | Gd | 4.1 | 4.9 | 0.14 |
| 175 | Example 129 | Gd | 10.3 | 11.6 | 0.38 |
| 176 | Example 130 | Gd | 27.0 | 10.1 | 0.20 |
| 177 | Example 131 | Gd | 21.4 | 10.1 | 0.19 |
| 178 | Example 132 | Gd | 12.6 | 12.5 | 0.40 |
| 179 | Example 133 | Gd | 14.7 | 10.8 | 0.18 |
| 180 | Example 134 | Gd | 11.4 | 9.4 | 0.25 |
| 181 | Example 135 | Gd | 6.0 | 11.2 | 0.58 |
| 182 | Example 136 | Gd | 3.9 | 9.8 | 0.46 |
| 183 | Example 137 | Gd | 6.5 | 23.2 | 1.08 |
| 184 | Example 138 | Gd | 4.9 | 5.2 | 0.21 |
| 185 | Example 139 | Gd | 3.9 | 18.2 | 1.00 |
| 186 | Example 140 | Gd | 10.4 | 5.6 | 0.19 |
| 187 | Example 141 | Gd | 11.9 | 5.8 | 0.17 |
| 188 | Example 142 | Gd | 4.8 | 9.5 | 0.21 |
| 189 | Example 143 | Gd | 13.0 | 9.1 | 0.15 |
| 190 | Example 144 | Gd | 10.0 | 7.9 | 0.08 |
| 191 | Example 145 | Gd | 10.0 | 8.4 | 0.15 |
| 192 | Example 146 | Gd | 24.0 | 9.2 | 0.19 |
| 193 | Example 147 | Gd | 7.0 | 8.6 | 0.17 |
| 194 | Example 148 | Gd | 13.3 | 18.1 | 0.25 |
| 195 | Example 149 | Gd | 23.0 | 20.4 | 0.29 |
| 196 | Example 150 | Gd | 9.2 | 9.5 | 0.19 |
| 197 | Example 151 | Gd | 9.5 | 8.8 | 0.17 |
| 198 | Example 143 | Mn | 9.4 | 7.4 | 0.15 |

[Comparative Example 1]

Preparation of magnescope solution

To PBS (4.6 mL), was added a Magnescope intravenous injection 38% syringes 10 mL (Guerbet Japan KK) (0.4 mL) to prepare a PBS solution in which Gd was 0.5 mol/L.

[Test Example 1] Measurement of relaxivity

The Gd-DOTA-bonded SCNP obtained in Example 152 was adjusted such that the Gd ion concentration in PBS was 1, 0.5, 0.25, 0.125, 0.0625, or 0.03125 mmol/L, respectively, and a longitudinal relaxation time ($T_1$ time) and a transverse relaxation time ($T_2$ time) were measured. From the obtained $T_1$ time and $T_2$ time, longitudinal relaxivity ($r_1$) and

transverse relaxivity ($r_2$) were given according to equations 1 and 2, respectively, and were 13.1 and 15.9.

The $T_1$ time and the $T_2$ time of the magnescope solution (Comparative Example 1) were measured by the same procedure as described above. From the obtained $T_1$ time and $T_2$ time, $r_1$ was 4.1, and $r_2$ was 4.9.

[Measurement apparatuses and conditions]

[Measurement apparatus]

Permanent magnet type 1 Tesla magnetic resonance imaging (MRI) apparatus (1.0 Tesla, ICON, Bruker Bisospin, Ettlingen, Germany)

[Conditions]

Longitudinal relaxivity measurement ($r_1$):
Inversion recovery spin-echo method
(RARE Sequence, TR/TE = 20 000/17 msec, RARE factor = 4, Inversion Time = 45, 100, 200, 400, 800, 1 600, 3 200, 6 400, 8 000, 10 000, 120 000 msec, FOV = 3.84 × 3.84 cm, Matrix Size = 64 × 64, Number of slice = 1, Slice thickness = 3 mm, NEX = 1, Scan time = 5 min 20 sec per scan)

[Conditions]

Transverse relaxivity measurement ($r_2$)

Multi-echo spin-echo method

(Reference measurement: MSME Sequence, TR = 15 000 msec, TE = 40, 80, 120, 160, 200 to 3 072 msec or 10 240 msec (256 step), FOV = 3.84 × 3.84 cm, Matrix Size = 64 × 64, Number of slice = 1, Slice thickness = 3 mm, NEX = 1, Scan time = 16 min 00 sec)

Formula 1 Definition of Relaxivity $r_1$

$$1/T_1 = 1/T_1^0 + r_1 \times [Gd]$$

In the equation, $T_1$ represents a longitudinal relaxation time(s) of water in the presence of the contrast agent, $T_1^0$ represents a longitudinal relaxation time(s) of water (in the absence of the contrast agent), $r_1$ represents the longitudinal relaxivity ($mS^{-1}s^{-1}$), and [Gd] represents a concentration (mmol/L) of Gd ions contained in the contrast agent.

Equation 2 Definition of Relaxivity $r_2$

$$1/T_2 = 1/T_2^0 + r_2 \times [Gd]$$

In the equation, $T_2$ represents a longitudinal relaxation time(s) of water in the presence of the contrast agent, $T_2^0$ represents a longitudinal relaxation time(s) of water (in the absence of the contrast agent), $r_2$ represents the transverse relaxivity ($mS^{-1}s^{-1}$), and [Gd] represents the concentration (mmol/L) of Gd ions contained in the contrast agent.

[Test Example 2] Contrast test

Tumor-bearing models obtained by subcutaneously transplanting a mouse colon cancer cell line C26 into female nude mice (BALB/c-nu/nu, 4 weeks old; Japan SLC, Inc.) were used for the contrast test.

The mouse colon cancer cell line C26 subcultured in a $CO_2$ incubator was suspended in a liquid medium (Dulbecco's Modified Eagle's Medium-high glucose, Sigma-Aldrich), and injected subcutaneously in the back of each nude mice such that the number of cells per mouse was $1 \times 10^6$/50 μL. Then, the nude mice were raised for about 10 days, and then administered with the agent when an average value of tumor volumes of the mice was grown to about 200 $mm^3$. The Gd-DOTA-bonded SCNP obtained in Example 152 was intravenously administered through the tail vein, and the contrast test was performed immediately after the administration, 1 hour after the administration, 2 hours after the

administration, and 24 hours after the administration. As a comparison, the magnescope solution (Comparative Example 1) was used, and administered in a similar manner. A dose of each agent was 0.4 mmol/kg in terms of Gd.

A temporal change in the imaging ability is shown in Fig. 12. In a case of the Gd-DOTA-bonded SCNP, an effect of enhancing the contrast was confirmed immediately after the administration, and the effect was sustained until 24 hours after the administration with a peak at 2 hours after the administration. Meanwhile, in a case of the magnescope solution (Comparative Example 1), an effect of enhancing the contrast was confirmed immediately after the administration, and the effect was reduced to the same extent as before the administration after 1 hour from the administration. Further, the effect of enhancing the contrast immediately after the administration of the magnescope solution was weaker than the effect of enhancing the contrast after 2 hours from the administration of the Gd-DOTA-bonded SCNP. The above results indicate that the Gd-DOTA-bonded SCNP has an excellent imaging effect compared to the magnescope solution.

[Measurement apparatuses and conditions]

[Measurement apparatus]

Permanent magnet type 1 Tesla magnetic resonance imaging (MRI) apparatus (1.0 Tesla, ICON, Bruker Bisospin, Ettlingen, Germany)

[Conditions]

T1-weighted image:

Spin echo method (MSME Sequence, TR/TE = 400/10 msec, FOV = 3.84 × 3.84 cm, Matrix Size = 256 × 256, Number of slice = 1, Slice thickness = 3 mm, NEX = 10, Scan time = 17 min 4 sec)

## Claims

1. A copolymer in which a chelating agent molecule is bonded to a copolymer X comprising structural units represented by the following formulas (A), (B), and (C):

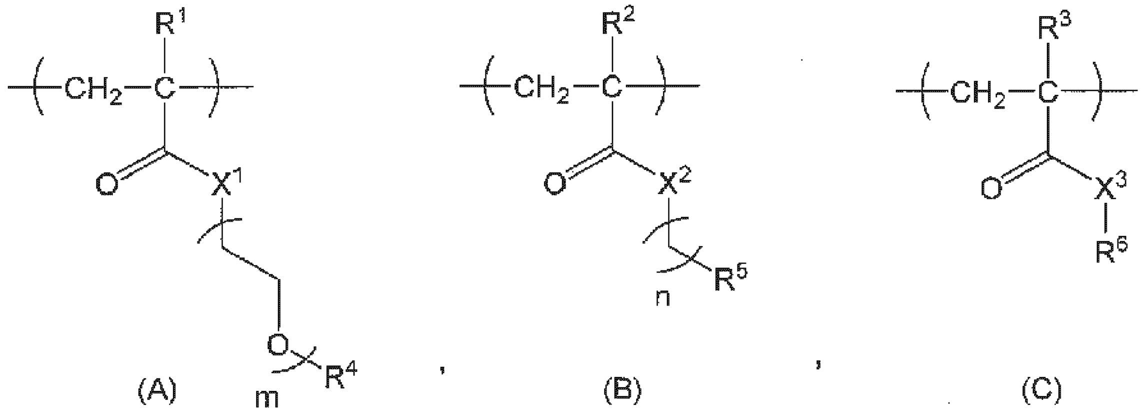

(A), (B), (C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8- membered cycloalkyl group optionally having a substituent, an adamantyl group, a $C_{6-18}$ aryl group optionally having a substituent, or a 5-to 10- membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

2. The copolymer according to claim 1, wherein the polymer X is a copolymer formed by polymerization of three types of monomers represented by the following general formulas (1) to (3):

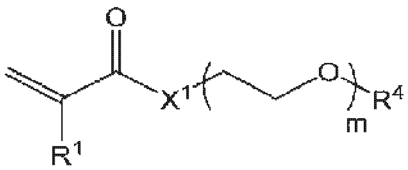 , 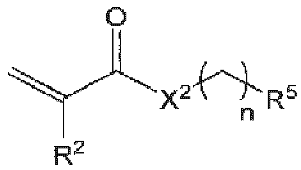 , 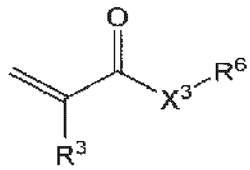

(1) (2) (3)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

**3.** The copolymer according to claim 1 or 2, wherein $R^1$ is a hydrogen atom.

**4.** The copolymer according to claim 1 or 2, wherein $R^2$ is a hydrogen atom.

**5.** The copolymer according to claim 1 or 2, wherein $R^3$ is a hydrogen atom.

**6.** The copolymer according to claim 1 or 2, wherein $R^4$ is a methyl group.

**7.** The copolymer according to claim 1 or 2, wherein $R^5$ is a $C_{6-18}$ aryl group optionally having a substituent.

**8.** The copolymer according to claim 1 or 2, wherein $R^5$ is a phenyl group.

**9.** The copolymer according to claim 1 or 2, wherein $R^6$ is a hydrogen atom.

**10.** The copolymer according to claim 1 or 2, wherein the leaving group of $R^6$ is a group represented by the following formula (4).

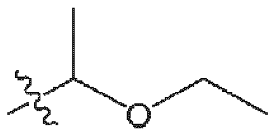

(4)

**11.** The copolymer according to claim 1 or 2, wherein the linker of $R^6$ is a group selected from the following formulas (5) to (7).

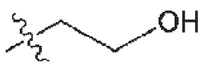 , 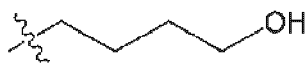 , 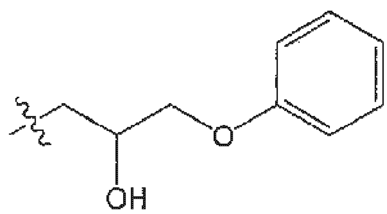

(5) (6) (7)

**12.** The copolymer according to claim 1 or 2, wherein $X^1$ is an oxygen atom.

**13.** The copolymer according to claim 1 or **2,** wherein $X^2$ is an oxygen atom.

**14.** The copolymer according to claim 1 or **2,** wherein $X^3$ is an oxygen atom.

**15.** The copolymer according to claim 1 or **2,** wherein m is an integer of 4 to 22.

**16.** The copolymer according to claim 1 or **2,** wherein n is 1.

**17.** The copolymer according to claim 1, wherein a ratio of the structural units (A), (B), and (C) is 0.01 to 100 parts by mass of (B) and 0.1 to 100 parts by mass of (C) with respect to 1 part by mass of (A).

**18.** The copolymer according to claim **2,** wherein 0.01 to 100 parts by mass of monomer (2) and 0.1 to 100 parts by mass of monomer (3) are polymerized with respect to 1 part by mass of monomer (1).

**19.** The copolymer according to claim 1 or 2, wherein the copolymer has a number average molecular weight of 5 000 to 150 000.

**20.** The copolymer according to claim 1 or 2, wherein the chelating agent molecule is a molecule having a residue represented by the following formula (a):

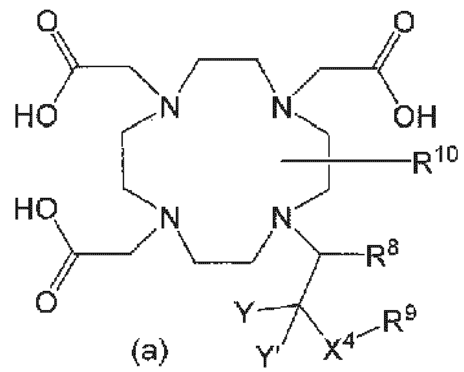

(a)

wherein, $R^8$ represents a hydrogen atom or a hydroxyalkyl group; $R^9$ and $R^{10}$ represent a hydrogen atom or $-[(CH_2)_o-L-(CH_2)_p]-*$; $X^4$ represents an oxygen atom, a sulfur atom, $N-R^7$, or $-CH_2-O-$; Y and Y' represent a hydrogen atom, a methyl group, or a hydroxy group, or Y and Y' together represent an oxygen atom; L represents an arylene group, a cycloalkylene group, -S-S-, -C(=O)O-, -OC(=O)-, -C(=O)NH-, -NHC(=O)-, -NHC(=O)O-, -OC(=O)NH-, or a peptide bond including 1 to 4 amino acid residues; * represents a bond to the copolymer; and o and p independently represent an integer of 0 to 10; where $R^9$ is a hydrogen atom, $R^{10}$ is - $[(CH_2)_o-L-(CH_2)_p]-*$, and where $R^9$ is $-[(CH_2)_o-L-(CH_2)_p]-*$, $R^{10}$ is a hydrogen atom.

**21.** The copolymer according to claim 1 or 2, wherein the chelating agent molecule is 1,4,7,10-tetraazacyclododecane-1,4,7,10-tetraacetic acid (DOTA), 1-(2-hydroxypropyl)-1,4,7,10-tetraazacyclododecane-1,2,3-triacetic acid (HP-DO3A), 10-[1,1,1-tris(hydroxymethyl)methyl]-1,4,7-triscarboxymethyl-1,4,7,10-tetraazacyclododecane (DO3A-butrol), 1,4,7,10-tetraazacyclododecane-1,4,7-triacetic acid-10-(2-thioethyl)acetamide (DO3A-Thiol), or S-2-(4-aminobenzyl)-1,4,7,10-1,4,7,10-tetraazacyclododecane-1,4,7,10-tetraacetic acid (DOTA-p-$NH_2$-Bn).

**22.** The copolymer according to claim 1 or 2, wherein binding of the chelating agent molecule to the copolymer X is a covalent bond or a non-covalent bond.

**23.** A polymer contrast agent comprising the copolymer according to claim 1 or 2 and a paramagnetic metal.

**24.** The polymer contrast agent according to claim 23, wherein the paramagnetic metal is gadolinium or manganese.

**25.** A diagnostic imaging drug comprising the copolymer according to claim 1 or 2 and a paramagnetic metal.

**26.** The diagnostic imaging drug according to claim 25, wherein the paramagnetic metal is gadolinium or manganese.

**27.** A single chain nanoparticle comprising the copolymer according to claim 1 or 2.

**28.** A pharmaceutical composition comprising the copolymer according to claim 1 or 2.

**29.** A single chain nanoparticle comprising the copolymer according to claim 1 or 2.

**30.** A single chain nanoparticle comprising the polymer contrast agent according to claim 23.

**31.** A single chain nanoparticle comprising the diagnostic imaging drug according to claim 25.

FIG. 1

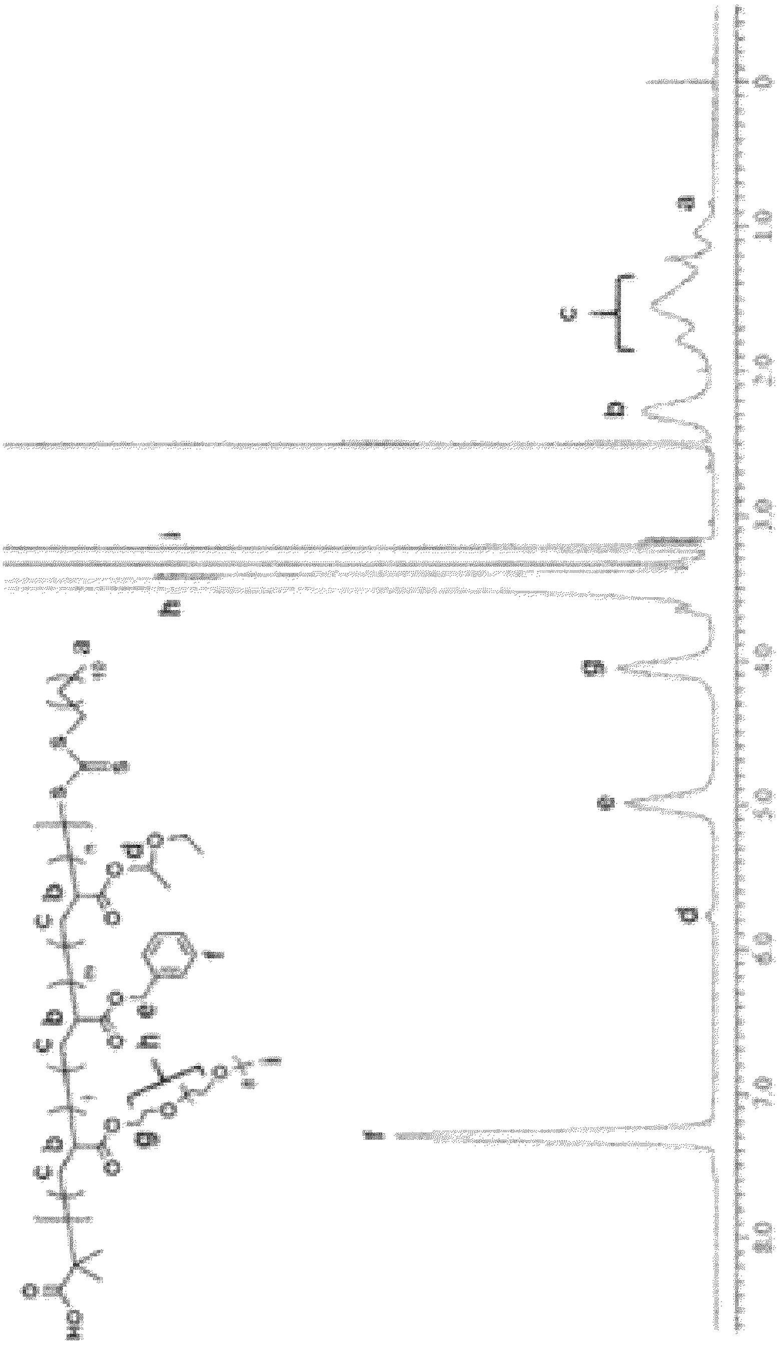

FIG. 2

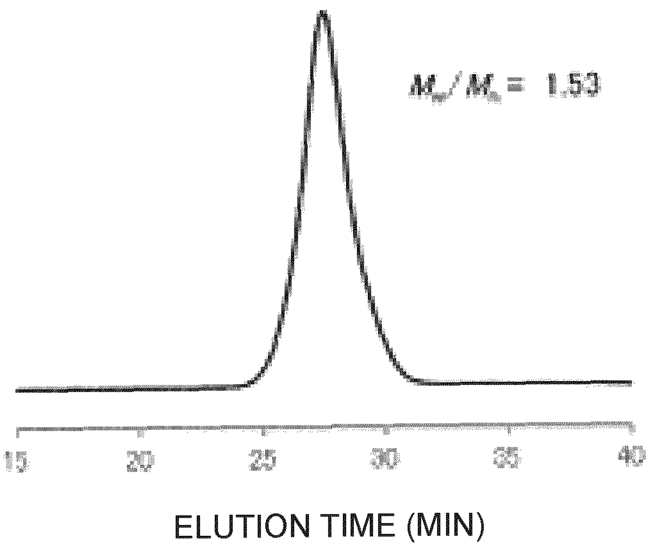

FIG. 3

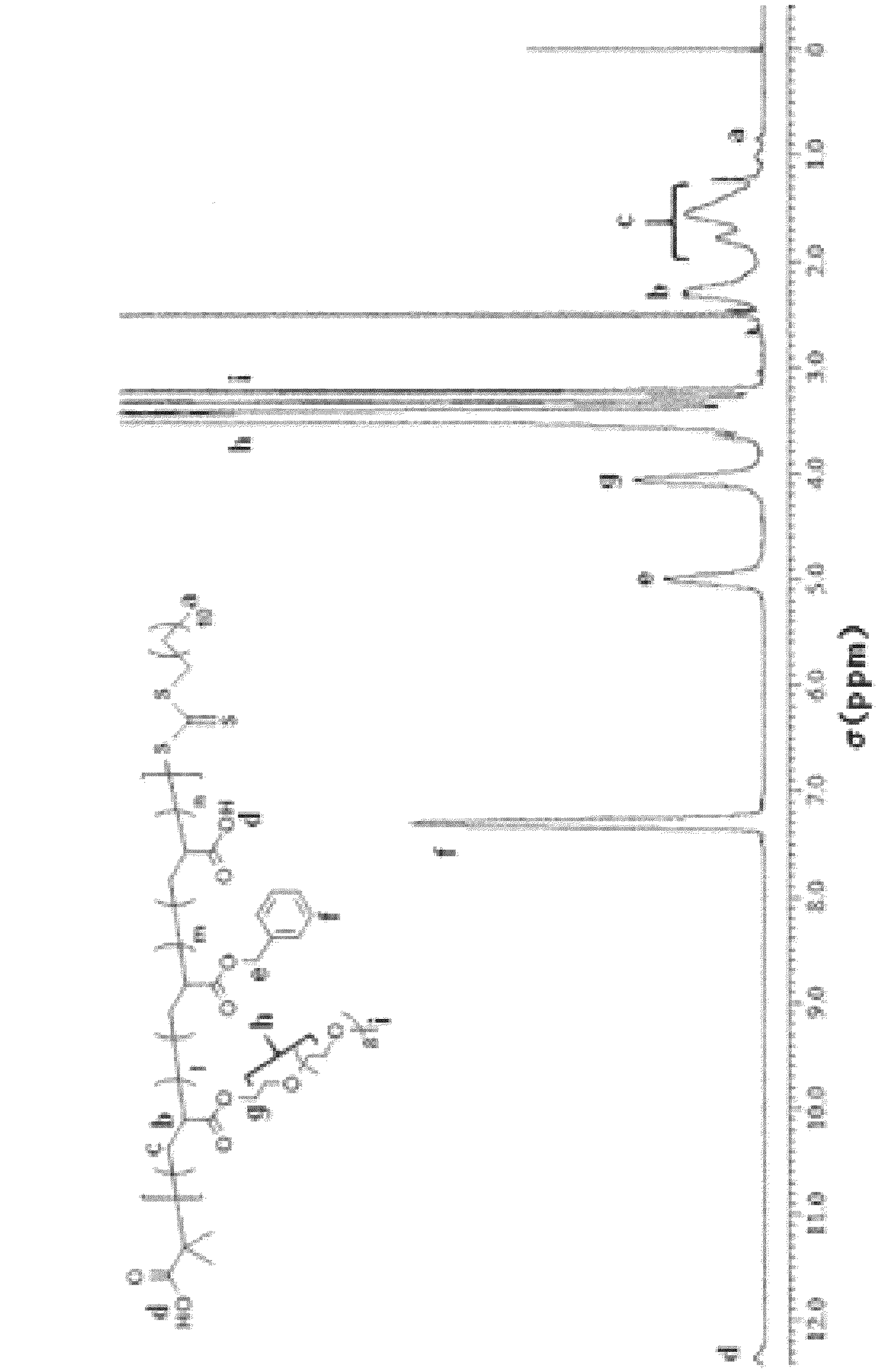

FIG. 4

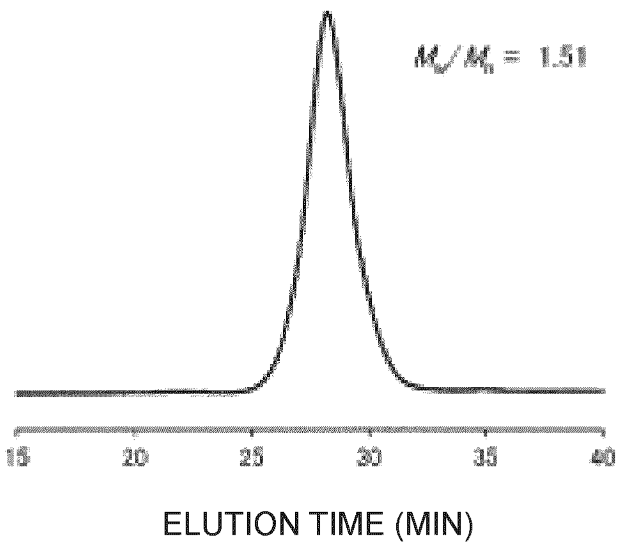
$M_w/M_n$ = 1.51
15
20
25
30
35
40
ELUTION TIME (MIN)

FIG. 5

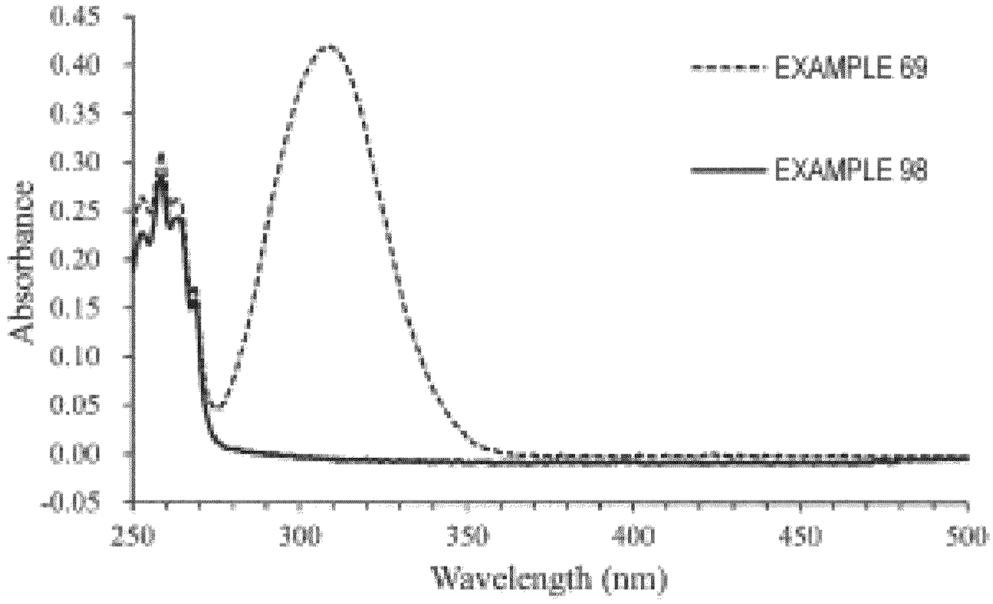
0.45
0.40
0.35
0.30
0.25
0.20
0.15
0.10
0.05
0.00
-0.05
Absorbance
EXAMPLE 69
EXAMPLE 98
250
300
350
400
450
500
Wavelength (nm)

FIG. 6

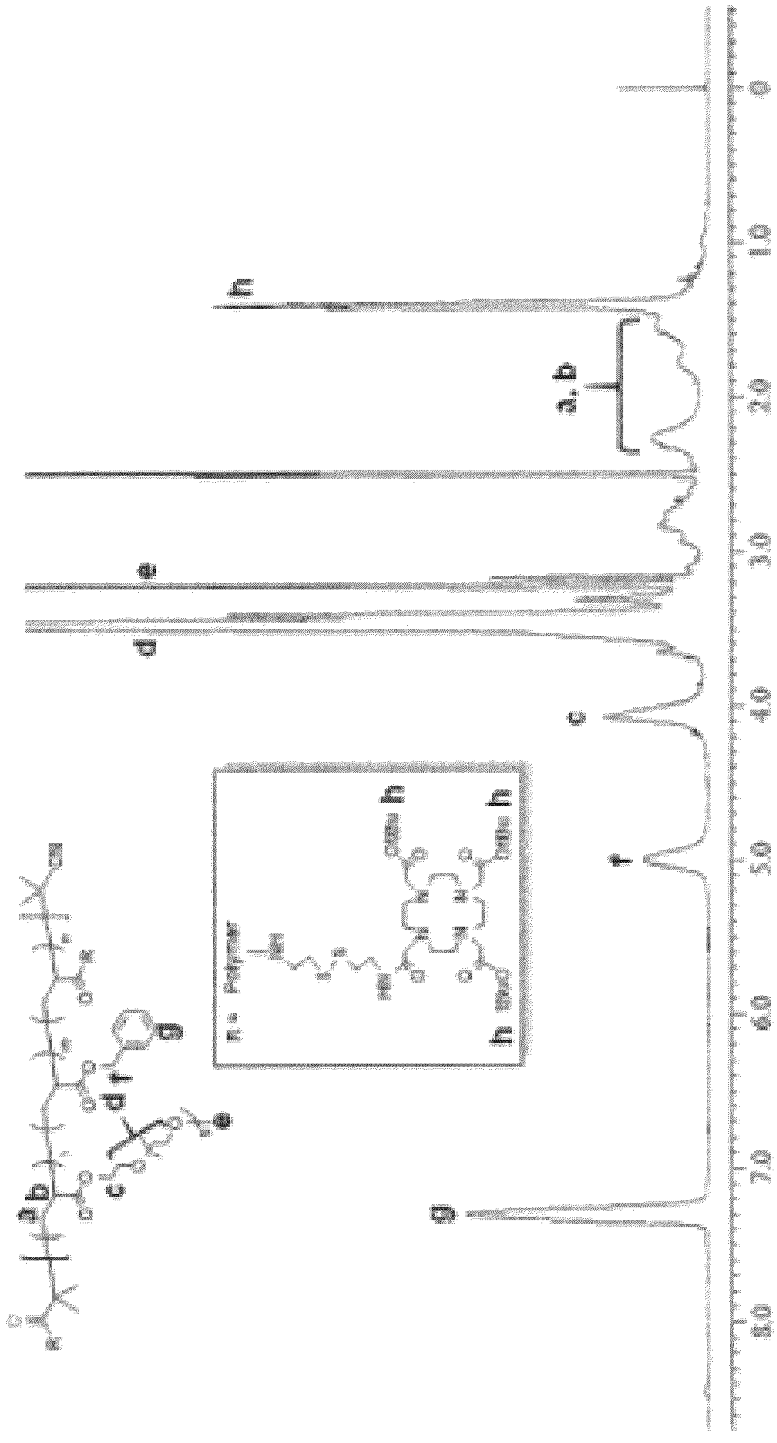

FIG. 7

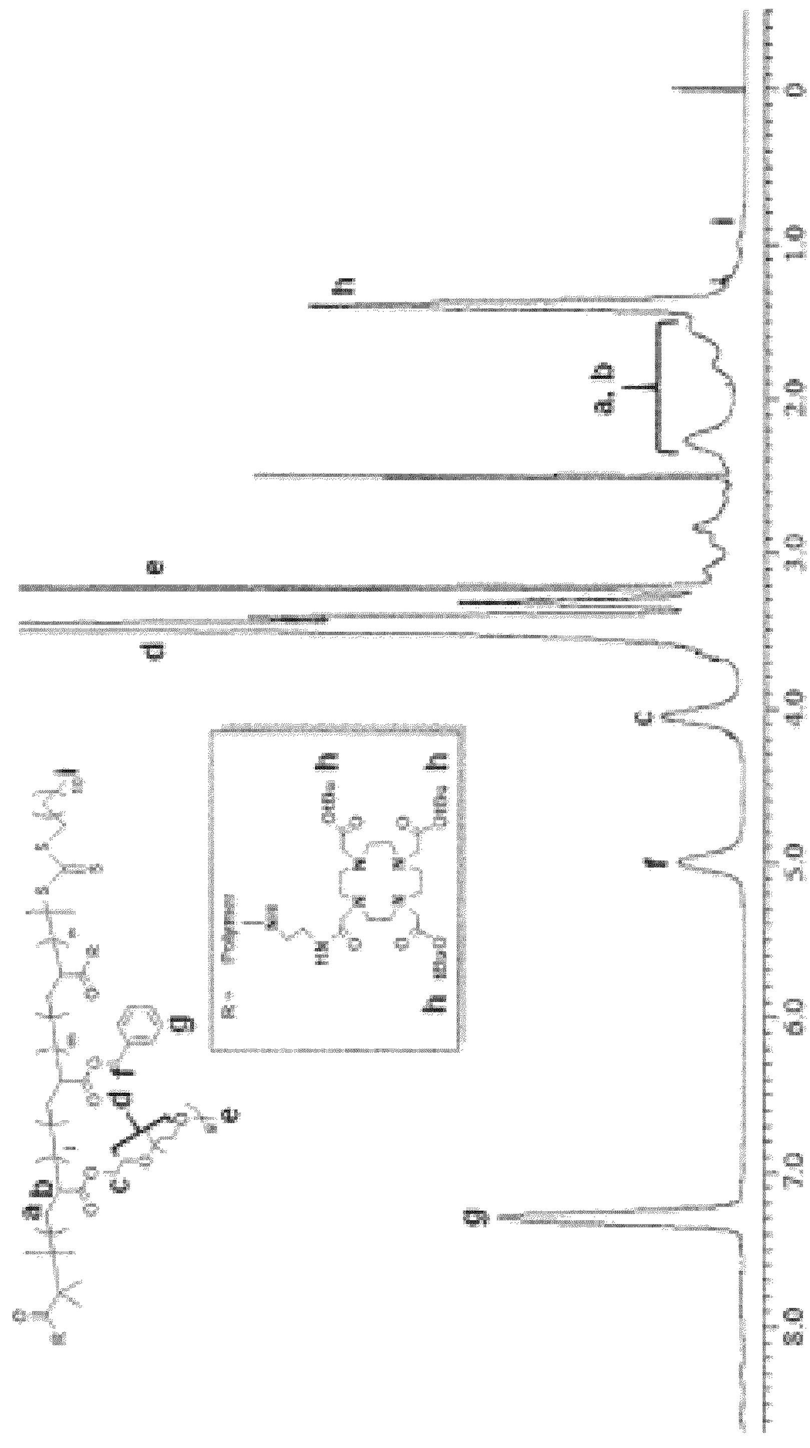

FIG. 8

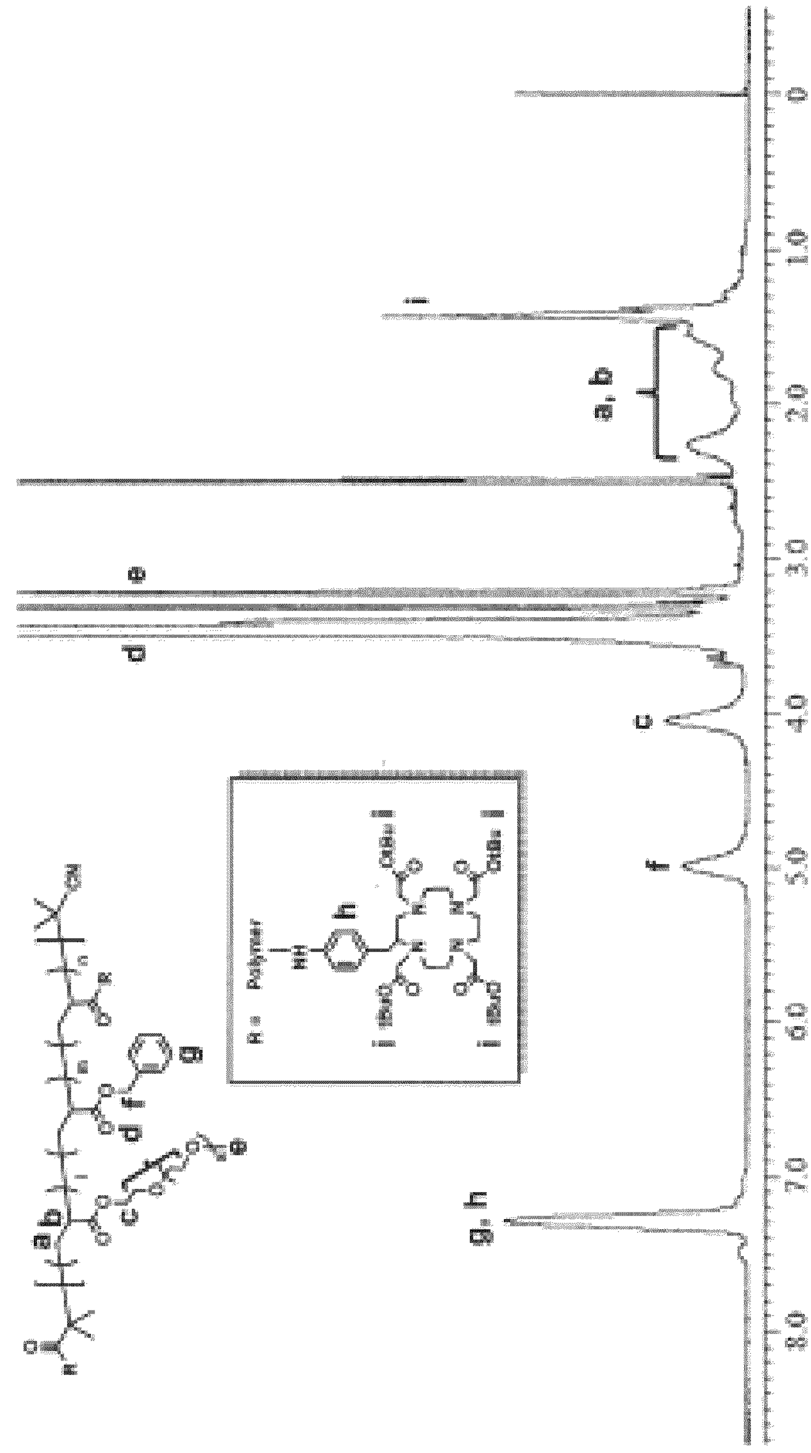

*FIG. 9*

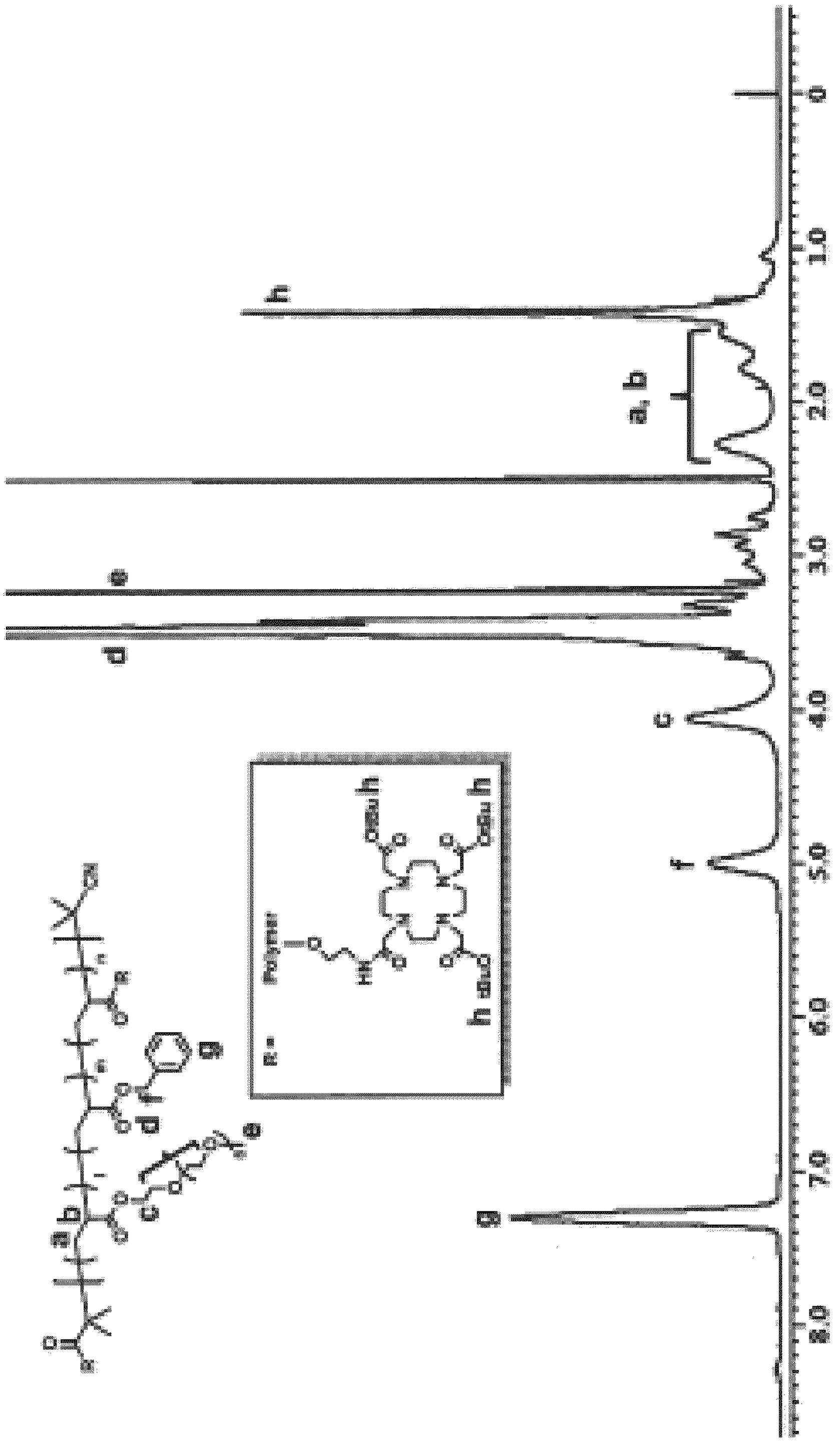

FIG. 10

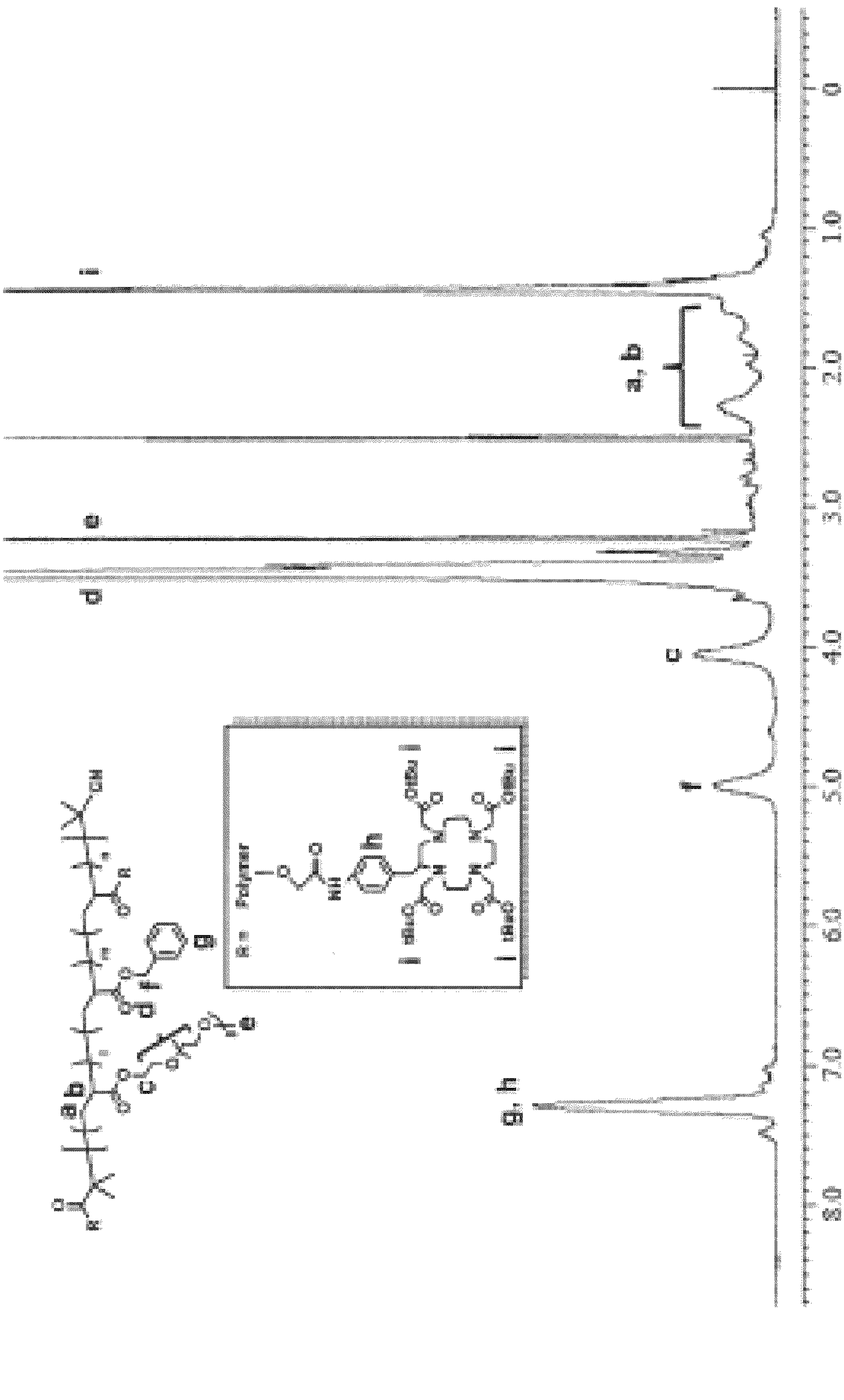

FIG. 11

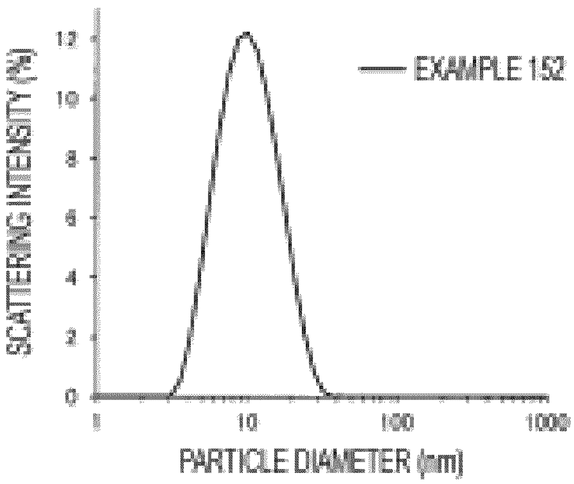
SCATTERING INTENSITY (%)
12
10
8
6
4
2
0
1
10
100
1000
PARTICLE DIAMETER (nm)
EXAMPLE 152

FIG. 12

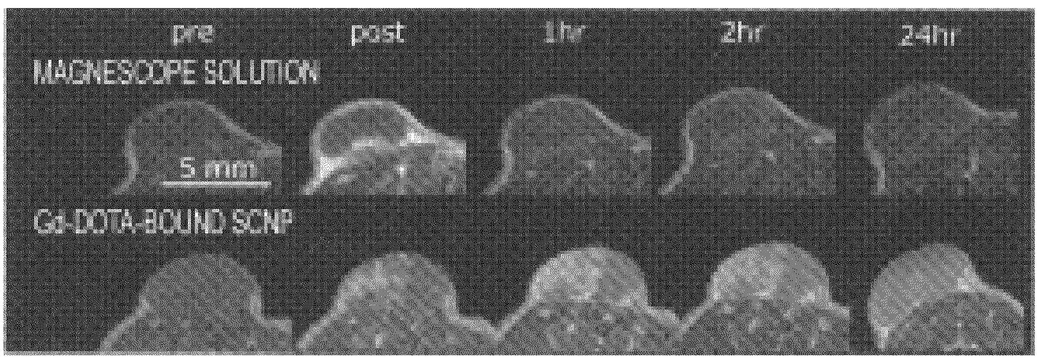
pre
post
1hr
2hr
24hr
MAGNESCOPE SOLUTION
5 mm
Gd-DOTA-BOUND SCNP

## INTERNATIONAL SEARCH REPORT

International application No.
**PCT/JP2023/029358**

**A. CLASSIFICATION OF SUBJECT MATTER**

***C08F 220/28***(2006.01)i; ***A61K 9/51***(2006.01)i; ***A61K 47/22***(2006.01)i; ***A61K 47/32***(2006.01)i; ***A61K 49/12***(2006.01)i; ***A61K 49/18***(2006.01)i; ***C07D 257/02***(2006.01)i
FI: C08F220/28; A61K9/51; A61K47/22; A61K47/32; A61K49/12; A61K49/18; C07D257/02

According to International Patent Classification (IPC) or to both national classification and IPC

**B. FIELDS SEARCHED**

Minimum documentation searched (classification system followed by classification symbols)

C08F220/28; A61K9/51; A61K47/22; A61K47/32; A61K49/12; A61K49/18; C07D257/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | MARSDEN, Catherine J. et al. Crosslinked p(MMA) particles by RAFT emulsion polymerisation: tuning size and stability. Polymer Chemistry. 01 June 2022, vol. 13, no. 28, pp. 4124-4135, DOI: 10.1039/d2py00337f<br>pp. 4130-4131, [Copolymerisation with MRI-active monomer], p. 4133, [Synthesis of p(OEGMEM)50-r-p(Gd・L1)0.5-b-p(OEGMEM)25-p(MMA)1000], fig. 1, 5 | 1-6, 9-31 |
| A | entire text | 7-8 |
| X | JACKSON, Alexander W. et al. Octreotide Functionalized Nano-Contrast Agent for Targeted Magnetic Resonance Imaging. Biomacromolecules. 16 November 2016, vol. 17, no. 12, pp. 3902-3910, DOI: 10.1021/acs.biomac.6b01256<br>abstract, p. 3907, left column, lines 13-16, scheme 1, fig. 1 | 1-6, 12-31 |
| A | entire text | 7-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2023** | **26 September 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2023/029358**

**C. DOCUMENTS CONSIDERED TO BE RELEVANT**

| Category* | Citation of document, with indication, where appropriate, of the relevant passages | Relevant to claim No. |
|---|---|---|
| A | PRIESTER, Aaron et al. Theranostic Copolymers Neutralize Reactive Oxygen Species and Lipid Peroxidation Products for the Combined Treatment of Traumatic Brain Injury. Biomacromolecules. 22 March 2022, vol. 23, no. 4, pp. 1703-1712, DOI: 10.1021/acs.biomac.1c01635<br>entire text | 1-31 |
| A | CN 104817664 A (ZHEJIANG UNIVERSITY) 05 August 2015 (2015-08-05)<br>entire text | 1-31 |

Form PCT/ISA/210 (second sheet) (January 2015)

**INTERNATIONAL SEARCH REPORT**
**Information on patent family members**

International application No.
**PCT/JP2023/029358**

| Patent document cited in search report | Publication date (day/month/year) | Patent family member(s) | Publication date (day/month/year) |
|---|---|---|---|
| CN 104817664 A | 05 August 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3270592 B
- JP 5883797 B
- JP 4892378 B


### Non-patent literature cited in the description


- **H. CABRAL et al.** *Nat. Nanotechnol*, 2011, vol. 6, 815-823
- **AVNESH S. THAKOR et al.** *J Nucl Med*, 2016, vol. 57, 1833-1837
- **JOSE A. POMPOSO**. *Single-Chain Polymer Nanoparticles: Synthesis, Characterization, Simulations, and Applications*, 2017
- Protective Groups in Organic Synthesis. John Wiley & Sons, Inc.